# EUROPEAN PATENT SPECIFICATION

(11) **EP 0 487 621 B1**
(45) Date of publication and mention of the grant of the patent: **02.10.1996**
(21) Application number: 90913261.5
(22) Date of filing: 17.08.1990
(51) Int. Cl.: C07K 17/02, C07K 16/00, G01N 33/68, G01N 33/72

(54) **IMMUNOASSAY OF GLYCOSYLATED PROTEINS EMPLOYING ANTIBODY DIRECTED TO REDUCTIVELY GLYCOSYLATED N-TERMINAL AMINO ACIDS**
TESTVERFAHREN VON GLYKOSYLIERTEN PROTEINEN UNTER VERWENDUNG EINES ANTIKÖRPERS GERICHTET GEGEN REDUZIERTE GLYKOSYLIERTE N-TERMINAL-AMINOSÄUREN
IMMUNOANALYSE DE PROTEINES GLYCOSYLEES UTILISANT UN ANTICORPS DIRIGE SUR DES ACIDES AMINES GLYCOSYLES A TERMINAISON N

(30) Priority: 23.08.1989 US 397781
(43) Date of publication of application: 03.06.1992
(73) Proprietor: NORTHWESTERN UNIVERSITY, Evanston Illinois 60208 (US)
(72) Inventor: ANDERSON, Byron, E., Morton Grove, IL 60053 (US); DAVIS, Lyman, E., Chicago, IL 60626 (US)
(74) Representative: Kügele, Bernhard
(86) International application number: US9004666
(87) International publication number: WO9102978

(56) References cited:
- EP-A- 0 201 187
- DE-A- 3 439 610
- US-A- 4 478 744
- US-A- 4 629 692
- US-A- 4 647 654
- US-A- 4 658 022
- Analytical Biochemistry, Volume 175, issued 1988. A. J. FURTH, "Methods for Assaying Nonenzymatic glycosylation", pp. 347-360, see page 358, left hand column.
- J. Clin. Pathol, Volume 37, issued 1984, I. PEACOCK, "Glycosylated haemoglobin: measurement and Clinical use", pp. 841-851, see page 841, Structure; page 842, the Table; and page 845, Radioimmunoassay.
- The Journal of Biological Chemistry, Volume 255, No. 7, issued 10 April 1980, R. SHAPIRO et al., "Sites of Nonenzymatic Glycosylation of Human Hemoglobin A", pp. 3120-3127, see page 3120.
- British Journal of Hematology, Volume 38, issued 1978, J. JAVID et al., "Immunologic Characterization and Quantification of Haemoglobin A1c", pp. 329- 337, see pages 329 and 336.
- Diabetes, Volume 30, issued 30 July 1981; F. BUNN, pages 613-617

## Description

### BACKGROUND OF THE INVENTION

This invention relates to a method of detecting and quantifying glycosylated proteins. More particularly, the invention relates to an immunoassay for detecting and quantitating proteins that are glycosylated on their amino-terminal (N-terminal) amino acid. One such protein is hemoglobin A_{1c} (HbA_{1c}), and the invention is particularly directed to immunoassays for detecting and quantitating HbA_{1c}. The invention also relates to antibodies specific for reduced glycosylated N-terminal amino acids that are used in the immunoassays of the invention and to immunogens and methods for making these antibodies.

The in vivo glycosylation (also called "glycation") of proteins may occur either enzymatically or non-enzymatically. In non-enzymatic glycosylation, a sugar is covalently coupled to the epsilon amino groups of available lysine residues or to the alpha amino group of accessible amino terminal (N-terminal) amino acids of the protein.

Non-enzymatic glycosylation of proteins with glucose proceeds in two stages. First, glucose combines with the amino group of the lysine or of the N-terminal amino acid to form an aldimine compound (a Schiff base). This reaction is reversible, and dissociation to unmodified protein and glucose readily occurs. Next, the aldimine intermediate is converted to a stable ketoamine derivative (1-deoxyfructose) by the Amadori rearrangement. Finally, over a period of weeks, the free carbonyl of the ketoamine derivatives forms crosslinks between the glycosylated protein and adjacent proteins, and the resulting aggregates are called advanced glycosylation products.

Non-enzymatic glycosylation occurs in normal mammals and to a much greater extent in diabetic patients. Patients afflicted with diabetes are incapable of metabolizing glucose in a conventional manner resulting in increased amounts of glucose in their blood and urine. In diabetic and normal individuals, glucose has been shown to bind non-enzymatically to the amino groups of many proteins, including hemoglobin, collagen, albumin, lens crystallins, fibrinogen, lipoproteins, ferritin, myelin, transferrin, and immunoglobulins, although such glycosylation occurs to a greater extent in diabetics than in normals.

The quantitative measurement of glycosylated proteins in diabetes is clinically important for two reasons. First, the measurement of glycosylated protein levels allows for the monitoring of blood glucose levels over an extended time period and allows the assessment of diabetic metabolic control. The time period over which mean blood glucose concentrations may be determined is largely dictated by the length of time a given assayed protein normally remains in the circulation. Since transferrin and human serum albumin are normally present in the circulation for a short to intermediate time (the half-life of transferrin is about 8 days, and the half-life of albumin is about 10-14 days), the measurement of the glycosylated forms provides a means of assessing short to intermediate glycemia. Hemoglobin is normally present in the circulation for a long time (the half-life of hemoglobin being about 2 to 3 months), and the measurement of glycosylated hemoglobin provides a means of assessing long term glycemia.

Second, non-enzymatic glycosylation has been implicated in the development of chronic diabetic complications, including accelerated cataract formation (due to advanced glycosylation products of crystallin lens protein) and atherogenesis (due to lipoprotein entrapment on arterial walls) contributing to the narrowing of blood vessels of the heart, brain, eyes, kidneys, and periphery. The measurement of glycosylated proteins and their advanced products may, therefore, be of prognostic value in the pathological sequalae involved in chronic hyperglycemia.

The main component of human hemoglobin (about 80-90% of the total hemoglobin) is HbAₒ. It has a tetrameric structure comprising two alpha and two beta chains. HbAₒ is not glycosylated on its N-terminal amino acids. Preparations of HbAₒ isolated by ion exchange chromatography have been shown to contain a small proportion of HbAₒ molecules glycosylated on the available epsilon amino groups of lysines.

HbA₁ₐ₁, HbA₁ₐ₂, HbA_{1b} and HbA_{1c} are minor components of human hemoglobin identical in structure to HbAₒ, except for the presence of a sugar residue covalently attached to the N-terminal valine residue of the beta chain. The sugar residues attached to the valine residues of HbA₁ₐ₁, HbA₁ₐ₂ and HbA_{1c} are fructose diphosphate, glucose-6-phosphate and 1-deoxyfructose, respectively. The sugar attached to valine in HbA_{1b} is not known.

About 4 to 5% of the total hemoglobin is HbA_{1c} in normal persons, but the amount is substantially higher in diabetes, generally being about 8 to 10%, but sometimes as high as 20%. Also, its concentration varies widely in relation to diabetic control.

HbA_{1c} is the most frequently measured glycosylated protein for clinical purposes, and a number of tests have been developed to measure it. See, e.g., Furth, Analytical Biochemistry, 175, 347-60 (1988); Peacock, J. Clin. Pathol., 37, 841-51 (1984); Miedema and Casparie, Ann. Clin. Biochem., 21, 2-15 (1984); and U.S. Patent No. 4,629,692. However, it is believed that only four methods are currently being used clinically: (1) affinity chromatography using m-aminophenylboronate columns; (2) cation exchange chromatography; (3) electrophoresis; and (4) isoelectric focusing.

Each of the four measurement techniques that are currently in clinical use suffers from one or more of the following disadvantages: relatively high cost per sample measured; lack of specificity in the analyte measured; sensitivity to slight variations in conditions such as ionic strength, pH and temperature; difficulty in standardization; lack of reproducibility; being time consuming and labor intensive; inability to automate; and difficulty in assaying many samples simultaneously. See U.S. Patent No. 4,629,692; Furth, Analytical Biochemistry, 175, 347-60 (1988); Peacock, J. Clin. Pathol., 37, 841-51 (1984).

Immunoassays for measuring glycosylated proteins, particularly HbA_{1c}, are known. However, it is believed that none are in clinical use for the measurement of HbA_{1c}. These various immunoassay techniques, and the antigens and antibodies used in them, will now be discussed.

First, European Patent Application No. 201,187 describes the preparation of monoclonal antibodies using purified HbA_{1c}. In particular, the application reports the preparation of monoclonal antibodies that preferentially bind to the glycated amino groups of hemoglobin, such as that of the N-terminal valine residues of the HbA_{1c} beta chains. In particular, a glycated heptapeptide whose sequence corresponds to the sequence of the N-terminus of the beta chain of HbA_{1c} inhibited the binding of one of these monoclonal antibodies to HbA_{1c}, whereas the non-glycated heptapeptide and the reduced glycated heptapeptide did not inhibit this binding. The patent teaches that these monoclonal antibodies can be used in known immunoassay techniques to quantitate HbA_{1c}. It should be noted that only 2 out of 320 hybridomas produced monoclonal antibody preferentially reactive with HbA_{1c}, suggesting that little antibody specific for HbA_{1c} is normally produced by immunized animals.

U.S. Patent No. 4,629,692 (Dean) teaches an immunoassay method for determining total non-enzymatically glycosylated proteins and protein fragments in a biological fluid. The immunoassay method employs an antibody which selectively recognizes and binds Amadori-rearranged glucose residues (i.e., 1-deoxy-D-fructosyl residues). The immunogen used to prepare the antibody is Amadori-rearranged glucose covalently bound to a carrier molecule. Polylysine is the preferred carrier, and the Amadori-rearranged glucose is preferably attached to the epsilon amino groups of the lysine residues. The immunogen is prepared by glycosylating the carrier non-enzymatically in vitro. A linker may be used between the Amadori-rearranged glucose and the carrier. The preferred linker is lysine. Other linkers including lysine analogs and amino-functionalized amino acids such as ornithine and hydroxylysine may be used.

The antibody prepared as described above can be used in a conventional immunoassay to quantitate total glycosylated proteins in a biological fluid such as serum. To determine the level of a specific glycosylated protein, such as HbA_{1c}, it must first be separated from the other non-enzymatically glycosylated proteins. For instance, the Dean patent teaches that HbA_{1c} can be separated by phenylboronate affinity chromatography and then quantitated using the disclosed antibody.

U.S. Patent No. 4,658,022 (the '022 patent) teaches the preparation of antibodies to a linear peptide epitope of a protein. The linear peptide epitope comprises from 2 to 15 amino acids of any portion (N-terminal, C-terminal or other portion) of the protein's sequence and may be modified with non-peptide groups such as carbohydrates. The linear peptide epitope is coupled to an immunogenic carrier for purposes of immunizing an animal. To perform the immunoassay, the antibodies are contacted with the protein which has been denatured sufficiently to expose, or increase the exposure of, the linear peptide epitope used for the preparation of the antibody. The use of monoclonal antibodies is preferred.

The '022 patent and U.S. Patent 4,647,654 (the'654 patent) teach the use, in the system described above, of a glycosylated peptide containing at least 2 amino acids, preferably 5 to 15 amino acids, of the N-terminal sequence of hemoglobin coupled to an immunogenic carrier to prepare an antibody. The '654 patent teaches that a linker which optimizes antigenicity and coupling properties may be used between the peptide epitope and the carrier. The linker may comprise one or more amino acids not found in the normal sequence of hemoglobin. Both patents teach that monoclonal antibodies can be produced in this manner which are specific for the glycosylated synthetic peptide and the corresponding epitope on the HbA_{1c} molecule when the HbA_{1c} molecule is denatured to expose the epitope. These antibodies do not cross-react with HbAₒ or with non-glycosylated peptides. The immunoassays used are conventional, except for the denaturation of hemoglobin. It should be noted that only 9 out of 200 hybridomas produced monoclonal antibody preferentially reactive with HbA_{1c}, suggesting that little antibody specific for HbA_{1c} is normally produced by immunized animals.

U.S. Patent No. 4,478,744 (Mezei et al.) teaches the preparation of antibodies to a protein using a peptide antigen, the amino acid sequence of which corresponds to a portion of the amino acid sequence of the protein. With respect to hemoglobin, the patent teaches the preparation of antibodies to glycosylated hemoglobin, specifically HbA_{1c}, using a peptide consisting of 4 to 10, preferably 7, amino acids, the sequence of which corresponds to the N-terminal sequence of the beta chain of hemoglobin. The peptide is glycosylated before or after being coupled to an immunogenic carrier protein or polypeptide. The peptide-carrier combination is used to immunize an animal, preferably one that does not normally produce HbA_{1c}. Mezei et al. teaches that the resultant antibodies are specific for HbA_{1c} and may be used in conventional immunoassays to quantitate HbA_{1c}. However, the '654 patent discussed above describes experiments showing that a polyclonal sheep antiserum produced according to the Mezei et al. method has no detectable specificity for HbA_{1c} in an ELISA assay even when affinity purified (see Example 8 of the '654 patent). Also see, European Patent Application 201,187 discussed above.

U.S. Patent No. 4,247,533 (Cerami et al.) and Javid et al., British Journal of Haematology, 38, 329 (1978) teach the preparation of antibodies to HbA_{1c}. The antibodies are produced by immunizing an animal, preferably one that does not normally form HbA_{1c}, with column purified human HbA_{1c}. The resulting antibody reacted equally well with HbAₒ and HbA_{1c} and was, therefore, absorbed repeatedly with HbAₒ. The absorbed antibody clearly distinguished HbAₒ from its glycosylated derivatives, but still cross-reacted slightly with human HbA₁ₐ and HbA_{1b} and with dog and mouse HbA_{1c}. It also showed strikingly less reactivity with NaBH₄ reduced HbA_{1c} than with unreduced HbA_{1c}. Also, certain reduced glycodipeptides, including reduced glycosylated valyl-histidine, failed to inhibit the reaction of the absorbed antibody with HbA_{1c}. The absorbed antibody was of low affinity and low titer. To overcome this problem, a specially modified radioimmunoassay (RIA) was employed. The '654 patent discussed above teaches that the reproducibility of this method is open to question (see column 2, lines 38-41, of the '654 patent.

Curtiss and Witztum, J. Clin. Invest., 72, 1427 (1983) describes the generation and characterization of six murine monoclonal antibodies that bind reduced glycosylated human plasma lipoproteins, but do not react with nonglycosylated or unreduced glycosylated plasma lipoproteins. The antibodies were prepared by immunizing mice with three injections of homologous low density lipoprotein (LDL) reductively glycosylated in the presence of glucose and sodium cyanoborohydride, followed by one injection of reductively glycosylated human LDL just before harvesting spleen cells to prepare the hybridomas. In competitive inhibition RIA, the dominant epitope recognized by these antibodies on reduced glycosylated LDL was identified as glucitol-lysine, the reduced hexose alcohol form of glucose conjugated to the epsilon amino group of lysine (glucitol-lysine completely inhibited the binding of each of the antibodies to reduced glysosylated LDL). Each of the six antibodies reacted with all reduced glycosylated proteins studied, including high density lipoprotein, albumin, hemoglobin and transferrin. The antibodies were also capable of identifying and quantitating glucitol-lysine residues on total plasma proteins and isolated lipoproteins of normal and diabetic individuals after reduction of the proteins with NaBH₄.

Witztum et al., Proc. Natl. Acad. Sci. USA, 80, 2757 (1983) describes the preparation of polyclonal antibodies by immunizing guinea pigs with homologous glycosylated or reductively glycosylated LDL. Immunization with reductively glycosylated LDL produced a high-titered antiserum that reacted with reductively glycosylated guinea pig LDL, but not with unreduced glycosylated LDL. Glucitol-lysine was a highly effective inhibitor of the binding of this antibody to reductively glycosylated LDL, as were other reductively glycosylated human proteins, including hemoglobin, albumin and transferrin. LDL glycosylated in the absence of a reducing agent was also immunogenic, although the antiserum was of lower titer and affinity. Homologous reductively glycosylated albumin was also immunogenic, and immunization with this compound produced an antiserum that reacted with reductively glycosylated albumin, but not with unreduced glycosylated albumin or reductively glycosylated LDL. All antibody activities were measured in a solid-phase RIA.

Nakayama et al., Clinica Chimica Acta, 158, 293-99 (1986) describes an RIA for glycated human serum protein using antiserum obtained by immunizing guinea pigs with reductively glycated human albumin. The antiserum was affinity absorbed on columns of native human serum albumin, and the absorbed antiserum recognized reductively glycated albumin and glucitol-lysine, but not non-reductively glycated albumin, native human albumin, lysine, sorbitol or mannitol. The antiserum was also capable of identifying and quantitating native human serum albumin and non-reductively glycated albumin after reduction of the protein with NaBH₄.

Go et al., Clinical Chimica Acta, 163, 63-73 (1987) reports the development of an enzyme linked immunosorbent assay (ELISA) for glycosylated proteins using a polyclonal antiserum which was prepared using reductively glycosylated homologous high and low density lipoproteins (HDL and LDL) as the immunogen. The article teaches that the antiserum is specific for the glucose-lysine bond, and that it recognizes, in a dose-dependent manner, all reduced glycosylated proteins tested, including albumin, fibrinogen, LDL, HDL, polylysine and hemoglobin. The antiserum had no affinity for native proteins or for unreduced glycosylated proteins. The ELISA assay is reported to be sensitive and capable of measuring a large number of samples in a relatively short period of time, but the article teaches that the conditions of the assay are critical and that major deviations from the described protocol lead to loss in sensitivity and reproducibility. See page 66 of Go et al.

### SUMMARY OF THE INVENTION

The present invention provides an immunoassay for a protein such as HbA_{1c} that is non-enzymatically glycosylated on the alpha amino group of its N-terminal amino acid. A high titered, high affinity antibody having specificity for Glc-ol-X is prepared as described below and used in the immunoassay. In the formula Glc-ol-X, X is the N-terminal amino acid of the glycosylated protein, and X may be any amino acid except lysine for the reasons discussed in detail below. Glc-ol is the reduced form of the sugar attached to X on the glycosylated protein.

A specific antibody, which is also part of the invention, is prepared by immunizing an animal with an immunogen of the formula (Glc-ol-X-L)ₙ-carrier wherein:

X is Valine and Glc-ol is as defined above, and Glc-ol is attached to the alpha amino group of X;
L is a bond or a linker group;
the carrier is an immunogenic compound other than the glycosylated protein; and
n is from 1 to the number of available coupling sites on the carrier.

The glycosylated protein that is to be assayed must be treated with a reducing agent before contacting it with the antibody to perform the immunoassay. This step is necessary to convert the glycosylated N-terminal amino acid of the protein to the Glc-ol-X form. In this way the antibody will recognize the glycosylated protein and bind with it. The antibody will recognize the glycosylated protein selectively in the presence of non-glycosylated proteins, proteins glycosylated on the epsilon amino group of lysine and proteins glycosylated on other N-terminal amino acids. However, if a plurality of proteins are present in a test sample which have the same glycosylated N-terminal amino acid, some method of identifying or separating out the protein of interest must be used.

The invention further comprises a specific (Glc-ol-Valine-L)ₙ-carrier compound used as the immunogen to prepare the antibody and methods of making this compound.

These methods comprise reductively glycosylating Valine or Valine-L to produce Glc-ol-Valine or Glc-ol-Valine-L. The Glc-ol-Valine and Glc-ol-Valine-L may then be directly coupled to the immunogenic carrier, or the Glc-ol-Valine may be coupled to L before being coupled to the carrier.

The invention also provides a kit for detecting or quantitating the glycosylated protein. The kit comprises a container of antibody directed to Glc-ol-Valine. The kit may also include one or both of the following: 1) a container of reducing agent for reducing the sugar residue on the N-terminal amino acid of the glycosylated protein or 2) a container of a labeled component useful for detecting or quantitating the glycosylated protein bound to the antibody.

### BRIEF DESCRIPTION OF THE DRAWINGS

Figures 1-3: A graph of relative fluorescence units (RFU) versus antibody dilution. The data on the graph were obtained by performing a direct binding enzyme linked fluorescence assay (ELFA) utilizing anti-glucitol-VGG-BSA antibodies which had been affinity purified on a column of glucitol-VGG Sepharose. The antigens are as indicated.

Figure 4: A typical graph of regression analysis results. The amount of HbA_{1c} detected by electrophoresis in lysates of red blood cell (RBC) from diabetics is on one axis, and the amount detected by direct binding ELFA utilizing anti-glucitol-VGG-BSA antibodies which had been affinity purified on a column of glucitol-VGG Sepharose is on the other axis.

Figure 5: A graph of RFU versus antibody dilution. The data on the graph were obtained by performing an ELFA utilizing anti-glucitol-VGG-BSA antibodies which had been affinity purified on a column of glucitol-valine Sepharose. The antigens are as indicated.

Figures 6-7: A graph of RFU versus antibody dilution. The data on the graph were obtained by performing an ELFA utilizing anti-glucitol-VGG-BSA antibodies which had been affinity absorbed with cross-linked human hemoglobin. The antigens are as indicated.

Figure 8: A graph showing the inhibition by reduced HbA_{1c} of the binding of affinity-purified anti-glucitol-VGG-BSA antibodies to reduced HbA_{1c}. The antibodies had been affinity purified on a column of glucitol-VGG Sepharose.

Figure 9: A graph showing the inhibition, by a reduced lysate of RBC's from a diabetic patient, of the binding of affinity-purified anti-glucitol-VGG-BSA antibodies to reduced HbA_{1c}. The antibodies had been affinity purified on a column of glucitol-VGG Sepharose.

Figure 10: A graph of regression analysis results. The amount of HbA_{1c} detected in lysates of RBC's from diabetics by electrophoresis is on the one axis, and the concentration of inhibitor necessary to cause a 50% inhibition of the binding of affinity-purified anti-glucitol-VGG-BSA to reduced HbA_{1c} in an inhibition ELFA assay is on the other axis. The inhibitor was reduced RBC lysates. The antibodies had been affinity purified on a column of glucitol-VGG Sepharose.

Figure 11: A graph showing the inhibition by glucitol-VGG of the binding of affinity-absorbed anti-glucitol-VGG-BSA antibodies to reduced HbA_{1c}. The antibodies had been affinity absorbed with cross-linked human hemoglobin.

Figure 12: A graph of regression analysis results. The amount of HbA_{1c} detected by electrophoresis in RBC lysates from diabetics is on one axis and the absorbance detected by direct binding ELFA utilizing DE-52 purified anti-glucitol-VGG-BSA and reduced whole blood lysates from the same diabetics is on the other axis.

Figure 13: A typical graph showing the inhibition by reduced whole blood lysates of the binding of DE-52 purified anti-glucitol-VGG-BSA antibodies to reduced HbA_{1c}.

Figure 14: A graph of regression analysis results. The amount of HbA_{1c} detected by electrophoresis in RBC lysates from diabetics is shown on one axis, and the amount of reduced whole blood lysate (expressed as total hemoglobin concentration in the lysate) from the same diabetics required to give 50% inhibition of the binding of DE-52 purified anti-glucitol-VGG-BSA antibodies to reduced HbA_{1c} is on the other axis.

Figure 15: A graph of regression analysis results. The amount of HbA_{1c} detected by the aminophenylboronate column method in RBC lysates from diabetics is on one axis, and the amount of HbA_{1c} detected in whole blood lysates from the same patients by direct binding ELISA utilizing DE-52 purified anti-glucitol-VGG-BSA antibody is on the other axis.

### DETAILED DESCRIPTION OF THE INVENTION

The immunogen used to stimulate production of the antibodies of the present invention comprises one or more Glc-ol-X-L residues coupled to an immunogenic carrier. X is the N-terminal amino acid of a protein that is glycosylated on the alpha amino group of its N-terminal amino acid. X may be any amino acid except lysine, and the glycosylated protein may be any such protein to which it is desired to produce an antibody, except those having lysine as the N-terminal amino acid.

The reason X cannot be lysine is as follows. Glycosylated proteins commonly contain lysine residues glycosylated on their epsilon amino groups. It is expected that antibodies formed to Glc-ol-lysine, where the Glc-ol is on the alpha amino group, would likely cross react with the more common glycosylated lysines having the sugar residue attached to their epsilon amino group. Thus, such antibodies would react with many different glycosylated proteins in a non-specific manner. The goal of the present invention is to avoid such a generalized non-specific reaction with numerous glycosylated proteins.

L in (Glc-ol-X-L)ₙ-carrier may be a bond linking Glc-ol-X to the carrier. If L is a bond, then X must terminate in a functional group such as carboxyl, thiol or hydroxyl which is active in a coupling reaction to an appropriate group in the carrier molecule.

L may also be any known linking group. For instance, L may be an aliphatic chain comprising from about 1 to about 20 atoms, excluding hydrogen. Normally the linking group will terminate in a functional group such as amino, carboxyl, thiol, hydroxyl or maleimido which is active in a coupling reaction to an appropriate group in the carrier molecule. It is most common to form amino or carboxyl derivatives and link them by conventional peptide condensation reactions to counterpart carboxyl and amino groups in the carrier.

Preferred linking groups are an amino acid or a peptide containing less than ten, preferably containing two, amino acids. The combination of X and the amino acid(s) of the linking group do not correspond to the N-terminal sequence of the glycosylated protein to which it is desired to form an antibody. The amino acid(s) of the linking group and the entire linking group are also preferably relatively non-immunogenic. For this reason, the amino acid glycine and the dipeptide glycine-glycine are particularly preferred linking groups.

When L is an amino acid or a peptide, it may be linked to X by known peptide synthetic methods. For instance, a solid phase peptide synthesis method like those described in Merrifield, JACS, 85, 2149 (1963); Davis et al., Biochemistry International, 10, 394-414 (1985); Stewart and Young, Solid Phase Peptide Synthesis (1969); U.S. Patent No. 3,941,763; Finn et al. in The Proteins, third edition, volume 2, pages 105-253 (Neurath et al. ed. 1976); and Erickson et al. in The Proteins, third edition, volume 2, pages 257-527 (Neurath et al. ed. 1976) may be used. These methods may also be used to prepare L when it is a peptide. Suitable synthetic peptides which may be used for X, for L when L is a peptide, or for X-L when L is an amino acid or peptide, may also be purchased commercially from various sources including Sigma Chemical Co., St. Louis, Missouri., Peninsula Laboratories, Belmont, California, Bachem Inc., Torrance, California and Vega Biochemicals, Tucson, Arizona.

It is preferred that X be reductively glycosylated before being coupled to the carrier to prevent the formation of Glc-ol-lysine residues or other Glc-ol residues that might produce interfering or non-specific antibodies. Further, if L is an amino acid or a peptide having epsilon amino groups, it is preferable to reductively glycosylate X before it is attached to L.

To reductively glycosylate X (alone or after being attached to L), an excess of a sugar corresponding to the one found on the N-terminal amino acid of the glycosylated protein of interest is reacted with X or X-L in the presence of a carbohydrate reducing agent such as sodium cyanoborohydride [see Curtiss and Witztum, Clin. Invest., 72, 1427-1438 (1983) and Friedman et al., Int. J. Pept. Protein Res., 6, 183-185 (1974)], pyridine-borane [see Wong et al., Anal. Biochem, 139, 58-67 (1984)], or sodium borohydride, [see Means and Feeney, Biochemistry, 7, 2191-2200 and Go et al., Proc. Nat'l Acad. Sci. USA, 80, 2751-2761 (1983)]. The reductively glycosylated X or X-L is separated from the carbohydrate reducing agent, and its non-glycosylated counterpart. This may be accomplished by conventional means, but preferably is accomplished using molecular sieve chromatography and, if necessary, ion exchange chromatography.

Suitable carriers are compounds capable of stimulating the production of antibodies to haptens coupled to them in a host animal. Such carriers are conventional and well-known. They are generally high molecular weight compounds. In most cases, the carrier will be a protein or polypeptide, although other materials such as carbohydrates, polysaccharides, lipopolysaccharides, nucleic acids, and the like of sufficient size and immunogenicity can be used.

Suitable immunogenic carrier proteins and polypeptides will generally have molecular weights between 4,000 and 10,000,000, and preferably greater than 15,000. Such suitable carriers include proteins such as albumins (e.g., bovine serum albumin, ovalbumin, human serum albumin), immunoglobulins, thyroglobulins (e.g., bovine thyroglobulin), hemocyanins (e.g., Keyhole Limpet hemocyanin) and polypeptides such as polylysine or polyalaninelysine.

Next the Glc-ol-X-L is coupled to the carrier. Methods of effecting this coupling are well-known. For instance, the Glc-ol-X-L residues may be coupled to the carrier with conjugating reagents such as glutaraldehyde, a water soluble carbodiimide such as 1-(3-dimethylaminopropyl)-3-ethylcarbodiimide hydrochloride (ECDI), N-N-carbonyldiimidazole, 1-hydroxybenzotriazole monohydrate, N-hydroxysuccinimide, n-trifluoroacetylimidazole cyanogen bromide, 3-(2'-benzothiazolyl-dithio) propionate succinimide ester, hydrazides, or affinity labeling methods. See also Pierce Handbook and General Catalog (1989) for a list of possible coupling agents.

Additional references concerning conventional immunogenic carrier materials and techniques for coupling haptens thereto are: Erlanger, Methods In Enzymology, 70, 85-104 (1980); Makela and Seppala, Handbook Of Experimental Immunology (Blackwell 1986); Parker, Radioimmunoassay of Biologically Active Compounds (Prentice-Hall 1976); Butler, J. Immunol. Meth., 7, 1-24 (1974); Weinryb and Shroff, Drug. Metab. Rev., 10, 271-83 (1979); Broughton and Strong, Clin. Chem., 22, 726-32 (1976); Playfair et al., Br. Med. Bull., 30, 24-31 (1974).

In the formula (Glc-ol-X-L)ₙ-carrier, n is the number of Glc-ol-X-L residues attached to the carrier. The number of such residues (the "epitopic density") on the carrier molecule will range from 1 to the number of available coupling groups on the carrier molecule. The epitopic density on a particular carrier will depend upon the molecular weight of the carrier and the density and availability of coupling sites. Optimal epitopic densities fall between about 10% and about 50% of the available coupling groups on the carrier molecule.

After the Glc-ol-X-L-carrier has been synthesized, it is used to prepare antibodies. Methods of preparing antibodies are well-known and conventional. For instance, the antibodies of the present invention may be prepared by injecting a suitable host animal (such as a rabbit, goat, horse or other mammal) with the immunogen of the invention in admixture with an adjuvant. The injections of immunogen are continued until an antiserum of suitable titer is obtained. The antiserum is harvested and may be further purified using known techniques if needed or desired. For instance, the antibodies may be affinity purified or may be fractioned such as by DE-52 chromatography.

Alternatively, the antibodies of the invention can be prepared by somatic cell hybridization by fusing cells from an immunized animal (such as rats, hamsters, mice or other mammal) with an immortal cell line such as myeloma cells. The fused cells are cloned, and monoclonal antibodies of appropriate specificity can be isolated by screening the cloned fused cells. Techniques of preparing monoclonal antibodies are well-known.

Thus, antibody suitable for use in the invention can be monoclonal or polyclonal antibody, can be an antiserum or a purified fraction thereof (such as DE-52 affinity purified or affinity adsorbed antibody), can be any of the known isotypes or subclasses (such as IgG, IgM, etc.), or can be an antibody fragment (such as Fab, F(ab') or F(ab')₂ that is capable of binding antigen. The only requirement is that the final antibody preparation have specificity for the Glc-ol-X epitope and be capable of binding to this epitope on the reduced glycosylated protein, the assay of which is desired.

The antibodies of the invention can be used in any immunoassay method that allows the detection or quantitation of non-enzymatically glycosylated proteins in a biological material. Many such techniques are known.

The only modification necessary is that the glycosylated protein that is to be assayed must be treated with a reducing agent before contacting the protein with the antibody to perform the immunoassay. This step is necessary to convert the glycosylated N-terminal amino acid of the protein to the Glc-ol-X form. In this way the antibody of the invention, which is specific for Glc-ol-X, will recognize the glycosylated protein and bind with it. No other special conditions are necessary. In particular, it is not necessary to treat the glycosylated protein with various denaturing conditions as is required by certain prior art assay techniques.

Suitable immunoassay methods include radioimmunoassay, enzyme immunoassay and fluorescence immunoassay. The immunoassay may be done in the competitive binding format or may be an immunometric assay. It may be a homogenous or heterogenous assay. Suitable homogenous techniques are fluorescence quenching and enhancement, energy transfer immunoassay, double antibody steric hinderance immunoassay, substrate-labeled immunoassay, prosthetic group-labeled immunoassay and enzyme modulator-labeled immunoassay.

One preferred immunoassay format is a direct binding assay in which reduced glycosylated protein in a sample such as a red blood cell (RBC) lysate is immobilized on a solid surface. Suitable solid surfaces are well-known and include glass, polystyrene, polypropylene, polyethylene, nylon, polyacrylamide, and agaroses. The immobilized antigen is contacted with antibody which is specific for Glc-ol-X (primary antibody). The primary antibody may be labeled so that the antibody bound to the immobilized antigen can be detected or quantitated. The amount of antigen (protein glycosylated on its N-terminal amino acid) in the sample can be quantitated since the amount of label bound to the solid surface after unbound materials are washed away is proportional to the amount of antigen present in the sample. Alternatively, after washing away unbound primary antibody, a labeled secondary antibody which binds specifically to the primary antibody may be added as a means to detect and quantitate the glycosylated protein of interest that is present in the sample.

Especially preferred direct binding immunoassays are the assays described in detail in Examples 10, 13 and 15. In particular, the direct binding assays described in Examples 13 and 15 employ lysates of whole blood, making the assays very simple to perform. They also utilize a colorimetric detection system. Such a system can be used to quantitate glycosylated proteins, but can also be used for a qualitative or semi-quantitative assay since the end point can be observed with the naked eye. Thus, such assays may be particularly valuable in settings where expensive equipment and experienced laboratory personnel are not available, such as in a physician's office, in a patient's home or in underdeveloped areas of the world.

Suitable labels for either the primary or secondary antibody are well-known in the art. They include: 1) enzymes (e.g., horseradish peroxidase, malate dehydrogenase, staphylococcal nuclease, delta-5-steroid isomerase, yeast alcohol dehydrogenase, alpha-glycerophosphate dehydrogenase, triose phosphate isomerase, alkaline phosphatase, asparaginase, glucose oxidase, beta-galactosidase, ribonuclease, urease, catalase, glucose-6-phosphate dehydrogenase, glucoamylase and acetylcholine esterase); 2) fluorophores (such as fluorescein isothiocyanate, rhodamine, phycoerythrin, phycocyanin, allophycocyanin, o-phtaldehyde and fluorescamine), 3) radionucleotides (such as¹²⁵I); 4) bioluminescent labels (such as luciferin, luciferase and aequorin); 5) chemiluminescent labels (such as luminol, isoluminol, aromatic acridinium ester, imidazole, acridinium salt and oxalate ester); and 6) biotin. The binding and detection of these labels can be done using standard techniques known to those skilled in the art.

An alternate and also preferred immunoassay construct is an inhibition assay. In this type of assay, varying amounts of liquid phase reduced glycosylated protein such as reduced HbA_{1c} in a RBC or whole blood lysate is used as the inhibitor. The inhibitor is mixed with and competes with a fixed amount of reduced glycosylated protein immobilized on a solid surface (such as those described above) for a limited number of available binding sites on the anti-Glc-ol-X antibody (primary antibody) By comparing the inhibition of primary antibody binding obtained for known inhibitor concentrations with that obtained with biological samples, the amount of glycosylated protein in the biological sample may be determined. The primary antibody may be labeled, or a labeled secondary antibody may be used. The labels are the same as described above. A particularly preferred inhibition assay is the inhibition ELFA described in Example 11.

A sandwich (capture, two-site) assay is also possible. In this assay, anti-Glc-ol-X antibody is immobilized on a solid surface such as those described above. The reduced glycosylated protein that is to be assayed is then contacted with the immobilized anti-Glc-ol-X antibody. After washing away unbound material, labeled antibody to the glycosylated protein is added, and the amount of labeled antibody bound is proportional to the amount of glycosylated protein in the original sample. The labels are the same as described above. The second labeled antibody does not have activity for Glc-ol-X, but is directed to another epitope on the glycosylated protein. Thus, this technique is particularly suitable for detecting or quantitating glycosylated protein when it is in admixture with other proteins that are glycosylated on the same N-terminal amino acid. Alternatively, the labeled antibody may be the antibody to Glc-ol, and the immobilized antibody may be the antibody directed to another epitope on the glycosylated protein.

Finally, a homogenous fluorescence polarization assay is possible. In such an assay, reduced glycosylated protein competes with Glc-ol-X-L or Glc-ol-X coupled to a fluorescent labeled low molecular weight carrier molecule (such as poly-L-lysine having a molecular weight of 3600) for the anti-Glc-ol-X antibody. The amount of fluorescence polarization is inversely related to the amount of reduced glycosylated protein in the test sample.

The specific concentrations, the temperature and time of incubation, as well as other assay conditions, can be varied in whatever immunoassay is employed depending on such factors as the concentration of the antigen in the sample, the nature of the sample, and the like. Those skilled in the art will be able to determine operative and optimal assay conditions for each determination while employing routine experimentation.

The biological material to be assayed according to the present invention may be any biological fluid containing the glycosylated protein of interest. It will usually be blood or a component thereof such as RBC lysates, serum or plasma, but may be other appropriate fluids such as saliva.

The invention also comprises a kit for detecting or quantitating a protein that is non-enzymatically glycosylated on the alpha amino group of its N-terminal amino acid. The kit is a packaged combination of one or more containers holding reagents useful in performing the immunoassays of the invention. Suitable containers for the reagents of the kit include bottles, vials, test tubes and microtiter plates.

The kit will comprise a container of antibody directed to Glc-ol-X. The antibodies are those describe above, and they may be labeled if they are to be used to detect or quantitate the glycosylated protein. The antibodies may be in solution, may be lyophilized or may be bound to a solid surface, such as those described above.

The kit may further comprise a container of reducing agent for reducing the sugar residue on the alpha amino group of the N-terminal amino acid of the glycosylated protein. These reducing agents are known and conventional and include those described above.

The kit may also comprise a container of a labeled component useful for detecting or quantitating the amount of the glycosylated protein bound to the antibody. This labeled component may be a labeled antibody specific for the anti-Glc-ol-X antibody, may be a labeled reduced glycosylated protein or peptide, or may be labeled Glc-ol-X or labeled Glc-ol-X-L such as Glc-ol-X or Glc-ol-X-L coupled to poly-L-lysine for fluorescence polarization.

Finally, the kit may contain other materials which are known in the art and which may be desirable from a commercial and user standpoint such as buffers, enzyme substrates, diluents, standards, etc. The kit may also include containers such as test tubes and microtiter plates for performing the immunoassay.

The invention is particularly directed to an immunoassay for glycosylated hemoglobin. More particularly, it is directed to an immunoassay for HbA_{1c} , and the invention provides a high titered, high affinity antibody that reacts specifically with glucitol-valine. A description of a preferred method of preparing such an antibody and of preferred immunoassay techniques which may be used to detect or quantitate HbA_{1c} is described in the Examples below, but other immunogens, antibody production techniques and immunoassay methods could be used as described in general terms above.

Briefly, in the preferred embodiment described below, the antibody was prepared by immunizing animals with the immunogen glucitol-valine-glycine-glycine-BSA. The resulting antisera were high titered with half-maximal binding to immunogens bearing glucitol-valine epitopes occurring at a dilution of 1:40,000 to 1:100,000.

The IgG fractions of the resulting antisera were prepared and affinity purified using either glucitol-valine-Sepharose or glucitol-valine-glycine-glycine-Sepharose. The resulting affinity purified antibody specifically detected the glucitol-valine epitope as shown by the results in Examples 10-12. In particular, the binding of the affinity purified antibody to reduced HbA_{1c} is specifically inhibited by reductively glycated HbA_{1c}, by reduced RBC lysates and by glucitol-valine-glycine-glycine. The approximate association constant (functional affinity) of the antibodies for HbA_{1c} is 1.5 to 3.4 nM.

It has also been determined that affinity purification or adsorption is not necessary to obtain an antibody preparation that reacts specifically with glucitol-valine epitopes. See Examples 13-15.

Finally, quantitative measurements of HbA_{1c} in RBC lysates and whole blood lysates correlate well with the results obtained by standard techniques. See Examples 10, 11 and 13-15.

### EXAMPLES

### EXAMPLE 1: Preparation of Glucitol-Valine-Glycine-Glycine

A reaction solution was prepared by dissolving 10 mg valine-glycine-glycine (VGG) peptide (Sigma Chemical Co., St. Louis, MO, USA) in 10 ml of an aqueous solution of 80mM glucose (Fisher Scientific Co., Fair Lawn, NJ, USA) and 12.5 mg/ml of freshly dissolved NaCNBH₃ (Sigma Chemical Co., St. Louis, MO, USA) prepared in distilled water. The freshly prepared reaction solution was filter sterilized using a 0.2 um acrodisc filter (Gelman Sciences, Ann Arbor, MI, USA) into a sterile screw cap 15 ml polystyrene tube (Corning Glass Works, Corning, NY, USA). Mock reaction, consisting of all reactants with the exception of glucose, was performed for comparative purposes.

The sterilized reaction solution was incubated at room temperature for 7-20 days, at which time reductively glycated VGG peptide was isolated by gel filtration chromatography using a 1 x 50 cm low pressure econo-column (Bio-Rad Laboratories, Richmond, CA, USA) containing a total volume of 40 ml of Bio-Gel P-2, 400 mesh (Bio-Rad Laboratories, Richmond, CA, USA), previously equilibrated with de-gassed distilled water. Fractionation was accomplished at ambient temperature by loading 1-2 ml of the reaction solution on the column. The column was run at a flow rate of 4 ml/hr, and 1 ml fractions were collected using distilled water as an eluting solvent.

The fractions collected were subsequently analyzed for peptide using bicinchonimic acid (BCA) [Smith P.K. et al., Anal. Biochem., 150, 76-85 (1985), Lane et al., J. Immunol. Methods, 92, 261-270 (1986)], or low U.V. absorption [Waddle, J. Lab. Clin. Med*.*, 48: 311-314 (1956)]. They were also analyzed for neutral hexoses using phenolsulfuric acid [Dische et al., Arch. Biochem. Biophys., 22: 169 (1949)], and for free amino groups using 2,4,6-trinitrobenzene sulfonic acid (TNBS) [Fields, Methods in Enzymology, 25b: 464-468 (1972)]. Peptide-containing fractions (BCA reactive) which were free of amino groups (no reaction with TNBS) and hexose (no reaction with phenol-sulfuric acid) were pooled, lyophilized, reconstituted with 1 ml of distilled water and re-analyzed by the BCA, TNBS and phenol-sulfuric acid methods.

The concentrated material remained BCA reactive, but failed to react with TNBS and phenol sulfuric acid, suggesting that the isolated material consisted of glucitol-N-terminal-blocked VGG peptide devoid of free glucose and NaCNBH₃. Amino acid analysis revealed the presence of glycine but not valine which is expected if all available valine residues were reductively glycated. The purified material was subsequently coupled to protein carriers and Sepharose 4B (see Examples 3-5).

### EXAMPLE 2: Preparation of Glucitol-Human Hemoglobin

Human hemoglobin (Sigma Chemical Co., St. Louis, MO, USA) was reductively glycated with glucose in the same manner as described above for VGG. Reductively glycated hemoglobin was separated from NaCNBH₃, glucose reactants and low-molecular weight reaction products by exhaustive dialysis against water using Spectropore cellulose dialysis tubing (American Scientific Products, McGaw Park, IL, USA) of an average molecular weight cut off of about 12-14K.

The extent of amino group modification was determined by the TNBS method of Fields, Methods in Enzymology, 25b, 464-468 (1972), using a control of human hemoglobin which had been reduced in the absence of glucose ("CNBH₃ control") as a measure of total available hemoglobin amino groups. Using this method, reductively glycated hemoglobin was determined to have 26% of available amino groups modified with glucitol. Glucitol-human hemoglobin so prepared was used in direct binding enzyme-linked fluorescence assay (ELFA) for antibody specificity characterization (see Example 10).

### EXAMPLE 3: Preparation of Glucitol-Valine-Glycine-Glycine-Bovine Serum Albumin (Glucitol-VGG-BSA)

Glucitol-valine-glycine-glycine (glucitol-VGG) prepared as described in Example 1 was coupled to bovine serum albumin (BSA) (Sigma Chemical Co., St. Louis, MO, USA) using 1-ethyl-3-(3-dimethylaminopropyl) carbodiimide hydrocholoride (EDCI) (Sigma Chemical Co., St. Louis, MO, USA) and the sodium salt of N-hydroxy-sulfosuccinimide (sulfo-NHS) (Pierce Chemical Co., Rockford, IL, USA) by a modification of the method of Staros et al., Anal. Biochem., 156, 220-222 (1986). Briefly, after pre-cooling all reactants on ice, 228 ul of a 10 mg/ml aqueous solution of sulfo-NHS was added to 500 ul of a 2.4 mg/ml aqueous solution of glucitol-VGG and mixed. Then, 1.5 ml of a freshly prepared 20 mg/ml solution of EDCI in distilled water was added, mixed immediately and allowed to react on ice for 15 minutes. BSA was subsequently added as 1160 ul of a 5 mg/ml aqueous solution. This reaction solution was mixed, and coupling was continued overnight at 4°C. The reaction mixture was then dialyzed exhaustively at 4°C against distilled water using Spectropore cellulose dialysis tubing (American Scientific Products, McGraw Park, IL, USA) of an average molecular weight cut-off of 12-14K. Controls in which distilled water was substituted for peptide, or for peptide and carbodiimide, or for peptide, carbodiimide and sulfo-NHS were included for assessment of both extent of conjugation and antibody specificity.

TNBS amino group determination, performed as described in Example 1, of glucitol-VGG-BSA conjugate and BSA conjugate control (without glucitol-VGG, EDCI or sulfo-NHS) revealed 42% of the 60 available BSA amino groups were substituted with glucitol-VGG after conjugation (i.e., there were 25 glucitol-VGG peptides/BSA carrier molecule).

### EXAMPLE 4: Preparation of Glucitol-Valine-Glycine-Glycine-Bovine Thyroglobulin (Glucitol-VGG-Thyro)

In a similar manner glucitol-VGG (prepared as described in Example 1) was coupled to bovine thyroglobulin (Sigma Chemical Company, St. Louis, MO, USA). Molar ratios of reactants were identical to those used for the coupling of glucitol-VGG to BSA described above in Example 3, except that an equimolar amount of 1-cyclohexyl-3-(2-morpholinoethyl)-carbodiimide metho-p-toluenesulfonate (CMC) was substituted for the EDCI as the water soluble carbodiimide.

TNBS amino group determination, performed as described in Example 1, of glucitol-VGG-Thyro conjugate and thyroglobulin conjugate control (without glucitol-VGG, CMC or sulfo-NHS) revealed that 26% of the estimated 194 available thyroglobulin amino groups were substituted with glucitol-VGG after conjugation.

### EXAMPLE 5: Preparation Of Glucitol-Valine-Glycine-Glycine-Sepharose

Glucitol-VGG-Sepharose immunosorbant was prepared by the addition of 254 mg of CMC to 533 ul of glucitol-VGG (8.67 mg/ml in H₂O). After mixing and solubilizing the carbodiimide, the peptide-carbodiimide solution was added to 2 ml of AH-Sepharose 4B (Pharmacia Fine Chemicals AB, Uppsala, Sweden) (prewashed according to manufacturer's instructions), and the mixture was mixed by inversion overnight at room temperature in a 4 ml (15x45 mm) screw cap micro sample vial (American Scientific Products, McGaw Park, IL USA). The immunoabsorbent was stored at 4°C in the presence of 0.02% NaN₃ prior to use. The immunoabsorbent was used in affinity purification as described in Example 7.

### EXAMPLE 6: Preparation of Glucitol-Valine-Sepharose

Glucitol-valine-Sepharose immunoabsorbent was prepared as described in Example 5, except that 595 ul of glucitol-valine (10 mg/ml in H₂O) were used in place of the glucitol-VGG. The glucitol-valine was prepared as described in Example 1 for VGG.

This immunoabsorbent was also stored at 4°C in the presence of 0.02% NaN₃ prior to use. It was used in affinity purification as described in Example 7.

### EXAMPLE 7: Preparation of Glucitol-Valine Reactive Antibodies

New Zealand white female rabbits (Langshaw Farms, Augusta, MI, USA) were initially immunized by the method of Vaitakaitis et al., Clin. Endo. Metab., 33, 988 (1971) with 2 ml of emulsion consisting of complete Freund's adjuvant containing 500 ug of glucitol-VGG-BSA prepared as described in Example 3. Subsequent immunizations were with emulsions of Freund's incomplete adjuvant containing 200 ug of glucitol-VGG-BSA. These subsequent immunizations were performed 5 weeks after the initial injection and at 2-8 week intervals thereafter.

The animals were bled from the ear 7-10 days after each immunization by the method of Nerenberg et al., J. Immol. Methods., 24, 19 (1978). Serum samples from each rabbit were examined for antibody reactivities by direct binding enzyme-linked immunofluorescence assay (ELFA) performed as described in Example 10 for all bleedings following the third immunization. Sera obtained were stored frozen at -20°C until used.

Purification of the desired antibodies was achieved by IgG fractionation of the sera using batchwise ion-exchange chromatography on DE-52 cellulose (Whatman LTD, Springfield Mill Maidstone Kent, England) by the method of Reif, Immunochemistry, 6, 723 (1969). With selected antisera, the IgG fractions were subsequently further purified by affinity chromatography on either glucitol-valine-Sepharose (prepared as described in Example 6) or glucitol-VGG-Sepharose (prepared as described in Example 5), or were affinity adsorbed using glutaraldehyde cross-linked human hemoglobin.

Affinity purification of anti-glucitol-VGG-BSA was accomplished by placing 1 ml of either glucitol-VGG-Sepharose or glucitol-valine-Sepharose in a Bio-Rad disposable polypropylene econo-column (Bio-Rad Laboratories, Richmond, CA, USA). The matrix was then washed with 50 ml of PBS (0.01M sodium phosphate in 0.15M NaCl), pH 7.3, followed by 3 ml of potassium iodide (KI) in PBS, pH 8.0, and then a 50 ml PBS, pH 7.3, wash. Subsequently, the washed matrix was incubated with rotation for 2 hours at 37°C with 14 ml of a DE-52 IgG fraction of antisera. Unbound material was collected, after which the matrix was washed two times with 10 ml PBS, pH 7.3, and bound antibody was collected by elution with 3 ml of 2M KI in PBS, pH 8.0, followed immediately by dialysis of the eluate against 2 liters of PBS, pH 7.3, overnight at 4°C. The resultant dialyzed affinity purified antibody was used in direct binding and inhibition ELFA assays (see Examples 10-11).

Removal of antibodies in the anti-glucitol-VGG-BSA preparation which might react with unreduced human hemoglobin was accomplished by affinity adsorbing DE-52 IgG fractions of antisera with denatured, glutaraldehyde-insolubilized human hemoglobin prepared as follows. Two hundred mg human hemoglobin (Sigma Chemical Co., St. Louis, MO, USA) dissolved in 10 ml of PBS, pH 7.3, containing 1M KI was cross-linked in water using a modification of the method of Avrameas and Terynck, Immunochemistry 6, 53 (1969) at a final glutaraldehyde concentration of 0.5%. After three days of reaction, insoluble hemoglobin was obtained by centrifugation, and was collected on a Whatman No. 1 paper (Whatman LTD, Springfield Mill Maidstone Kent, England) by vacuum filtration using a Coors-Buckner funnel (American Scientific Products, McGaw Park, IL, USA). The cross-linked hemoglobin on the filter was washed with 1 liter of PBS, pH 7.3. It was then incubated for 15 min at room temperature with 500 ml of 0.05 M NH₄Cl, which was removed by vacuum filtration. Next, the insolubilized hemoglobin was washed with 400 ml PBS, pH 7.3, which was also removed by vacuum filtration.

The washed cross-linked hemoglobin was used to remove any possible hemoglobin-reactive antibodies from anti-glucitol-VGG-BSA IgG fractions by incubating 10 mg of glutaraldehyde cross-linked human hemoglobin with 2 ml of a DE-52 fraction of antisera with mixing end over end on a rotator for 3.5 hours at 37°C. Hemoglobin-adsorbed, DE-52 IgG antibody not bound to the insolubilized hemoglobin was separated from insoluble hemoglobin and any material bound thereto using a Pasteur pipette and employed in direct binding ELFA assays (see Example 10).

### EXAMPLE 8: Preparation of Human Hemoglobin A_{1c} (HbA_{1c}) and Human Hemoblobin Aₒ(HbAₒ)

Human HbA_{1c} and HbAₒ were purified from outdated blood from a local blood bank. The red blood cells (RBC's) obtained by centrifugation were washed with PBS, pH 7.3, and lysed with 20 parts of distilled water. The residual stroma and cells were removed by centrifugation, and the lysate obtained was subjected to either a modification of the ion exchange-gel filtration method of Lowrey and Soeldner, Anal. Biochemistry, 154, 424-430 (1986), or to the ion exchange method of McDonald et al., J. Biol. Chem., 253, 2327-2332 (1987). The method of Lowrey and Soeldner was modified by increasing the column volume (2.5 x 42 cm), increasing the salt concentration in the lysate loaded (100 mM NaCl), decreasing the flow rate (15 ml/hr) and decreasing the volume of heme protein loaded (0.5 ml of lysate concentrated 10 fold). Although high purity HbA_{1c} and HbAₒ were obtained by the mixed bed procedure, the method of McDonald et al. was preferred due to its reproducibility.

Hemoglobin-containing fractions obtained by either isolation technique were identified by inspection and by absorption at 415 nm. The purity of the various hemoglobin fractions was assessed by analytical cation exchange HPLC using a 4.6 x 30 mM microanalyzer MA7C cartridge (Bio-Rad Laboratories, Richmond, CA, USA) in a Beckman 344 HPLC system (Beckman Instruments, Berkeley, Ca.). Hemoglobin fractionation was performed according to the manufacturer's suggested protocol (Bio-Rad Laboratories, Richmond, CA, USA). Briefly, 20 ul of a hemoglobin-containing sample was injected on the microanalyzer MA7C cartridge previously equilibrated with 20mM Bis-Tris, pH 6.0, and hemoglobin fractionation was obtained by the use of a linear salt gradient (0 to 100 mM NaCl) in the same Bis-Tris buffer system over 6 minutes at a flow rate of 1.5 ml/min. Hemoglobin containing fractions were detected by heme absorption at 415 nm using a Beckman 163 variable wavelength detector (Beckman Instruments, Berkeley, CA, USA). Hemoglobin fractions were detected by absorption at 415 nm and compared to standards containing known amounts of human hemoglobin A_{1c} (Bio-Rad Clinical Division, Hercules, CA, USA). Purified hemoglobin fractions were stored at 4°C until used in direct binding or inhibition ELFA assays (see Examples 10-12).

### EXAMPLE 9: Reduction of Hemoglobin Antigens

To be able to detect and quantitate the HbA_{1c}, if any, present in hemoglobin-containing samples (e.g., purified hemoglobin, hemoglobin components or RBC lysates from patients), the hemoglobin-containing sample must first be reduced with NaBH₄ to form a glucitol-valine on the N-terminal of the beta chains of any HbA_{1c} present in the sample. Reduced HbA_{1c} present in the sample can then react with anti-glucitol-VGG-BSA antibodies present in antisera, in DE-52 IgG fractions of antisera, and in affinity purified and affinity adsorbed materials (all prepared as described in Example 7). Reduction of solid-phase adsorbed hemoglobin is required for detection of glucitol-valine in direct binding ELFA, and reduction of both solid-phase and liquid-phase hemoglobin is required for inhibition ELFA assays (see Examples 10-14). The reduction of solid-phase adsorbed and liquid-phase hemoglobin will be described separately.

Hemoglobin previously adsorbed to polystyrene microtiter wells (see Examples 10-14) was reduced by the addition of 50 mM NaBH₄ in PBS, pH 7.3. The volume of reducing solution was equal to that used for hemoglobin coating. After reaction overnight at 4°C, the plates were washed 5 times by filling and decanting or aspirating the wells with PBSA (0.01 M sodium phosphate buffer containing 0.15 M NaCl, 1% BSA, 0.1% Tween 20, 0.02% NaN₃, pH 7.3) to remove residual borohydride. The plates were then processed in the manner described in Example 10 by back coating using 1% (wt/vol) ovalbumin in PBS, pH 7.3.

Reduction of liquid-phase hemoglobin samples was accomplished bY adding 3 volumes of 50 mM NaBH₄ in PBS containing 0.01% BSA, pH 7.3 ("reducing solution"), to each volume of hemoglobin solution to be reduced. The suggested concentration of unreduced hemoglobin using this reduction method is 0.5-1.0 mg/ml as determined by heme adsorption at 415 nm. The hemoglobin was mixed with the reducing solution by vortexing gently, and was then reacted for 3 hours at 37°C, at which time the reduction mixture was diluted 1:10 using PBSA. This dilution lowers the NaBH₄ concentration to 3.75 mM NaBH₄, a level determined not to interfere with antibody reactivity, and the diluted, reduced material was ready to be employed as the inhibitor in inhibition ELFA (see Example 11).

NaBH₄ reduction of hemoglobin obtained from red blood cell (RBC) lysates was performed in a similar manner to that described above for purified HbA_{1C} or HbAₒ. RBC's washed with physiological saline were lysed by diluting them 1:20 in distilled water. Insoluble debris was removed by settling or centrifugation, and the hemoglobin concentration of collected soluble supernatant lysate was determined. Reduction of solid-phase adsorbed lysate was performed as described above for HbA_{1c}. Reduction of liquid-phase lysate differed from the method described above only in the use of an initial concentration of 2 mg/ml of unreduced hemoglobin instead of 0.5-1.0 mg/ml.

The use of whole blood lysates (Examples 13 and 14) required variations in the reduction protocol which are described in detail in those examples.

### EXAMPLE 10: Direct Binding Enzyme Linked Fluorescence Assay ("Direct Binding ELFA")

Two hundred microliters of antigen (e.g., glucitol-VGG-BSA, glucitol-VGG-Thyro, glucitoi-human hemoglobin, CNBH₃ control, HbA_{1c}, HbAₒ, or lysates of RBC's from diabetic patients prepared as described in Examples 1-6 and 8-9) were added to wells of Microfluor "B" black polystyrene microtiter plates (Dynatech Laboratories, Alexandria, VA, USA). The plates were incubated for 2 hours at 37°C to allow the antigen to adsorb to the wells. The antigens were added at a coating concentration of 5 ug/ml protein in 0.1 M NaHCO₃, pH 9.8.

Unbound antigen was removed by decanting or aspirating, and NaBH₄ reduction was performed as described in Example 9 on adsorbed antigens requiring preliminary reduction (i.e., HbA_{1c}, Hbₒ and RBC lysates). The plates were washed 5 times with PBSA by filling the wells with PBSA and decanting or aspirating to remove residual NaBH₄. The reduction and PBSA washing steps were omitted for antigens already bearing glucitol.

Remaining polystyrene protein binding sites were subsequently blocked by filling wells with PBS containing 1% ovalbumin (Sigma Chemical Co., St. Louis, MO, USA) and 0.02% sodium azide, pH 7.3, and incubating for 1 hour at 37°C. After decanting or aspirating, 200 ul of anti-glucitol-VGG-BSA antibody preparation (e.g., antiserum, DE-52 IgG fraction, affinity purified antibody, or affinity adsorbed antibody, all prepared as described in Example 7) serially diluted in PBSA were added to wells and incubated overnight at 4°C in a humidified chamber.

The microtiter wells were next washed 5 times with PBSA as described above, and 200 ul/well of biotin labeled goat anti-rabbit IgG (Vector Laboratories, Burlingame, CA, USA) diluted 1:2000 in PBSA were added and allowed to incubate for 2 hours at 37°C. Following five PBSA washes, 200 ul/well of streptavidin-β-galactosidase (Bethesda Research Laboratories, Gaithersburg, MD, USA) diluted 1:2000 in PBSA were added and allowed to incubate 1.5 hour at 37°C. After a final five washes with PBSA, 200 ul of 0.1 mg/ml 4-methyl-umbelliferyl-8-D-galactopyranoside substrate in PBS, pH 7.5, were added to the wells of the microtiter plate, and methylumbelliferone fluorescence was measured as relative fluorescence units (RFU) using an excitation wavelength of 365 nm and an emission wavelength of 450 nM in a MicroFLUOR reader (Dynatech Instruments, Torrance, Ca. USA).

Control wells which were treated in an identical manner as described above but lacked only antigen ("without antigen controls"), lacked only anti-glucitol-VGG-BSA antibody preparation ("without primary antibody controls") and those that received only a blocking step and substrate ("substrate blank") were included. Without antigen controls and without primary antibody controls served to correct readings for non-specific binding of antibody and labeling reagents, while substrate controls corrected for non-enzymatic substrate hydrolysis.

The above protocol was modified slightly for direct binding assay of RBC lysate preparations. The alterations for RBC lysates were: the use of a coating concentration of 10 ug/ml protein with no subsequent blocking step; the use of 3 PBS, pH 7.3, washes instead of 5 PBSA washes; incubation of all reagents at ambient temperature for 1 hour, except for a 0.5 hour incubation with streptavidin-β-galactosidase; and the use of anti-glucitol-VGG-BSA antibody preparation at a fixed saturating concentration (1:100 dilution).

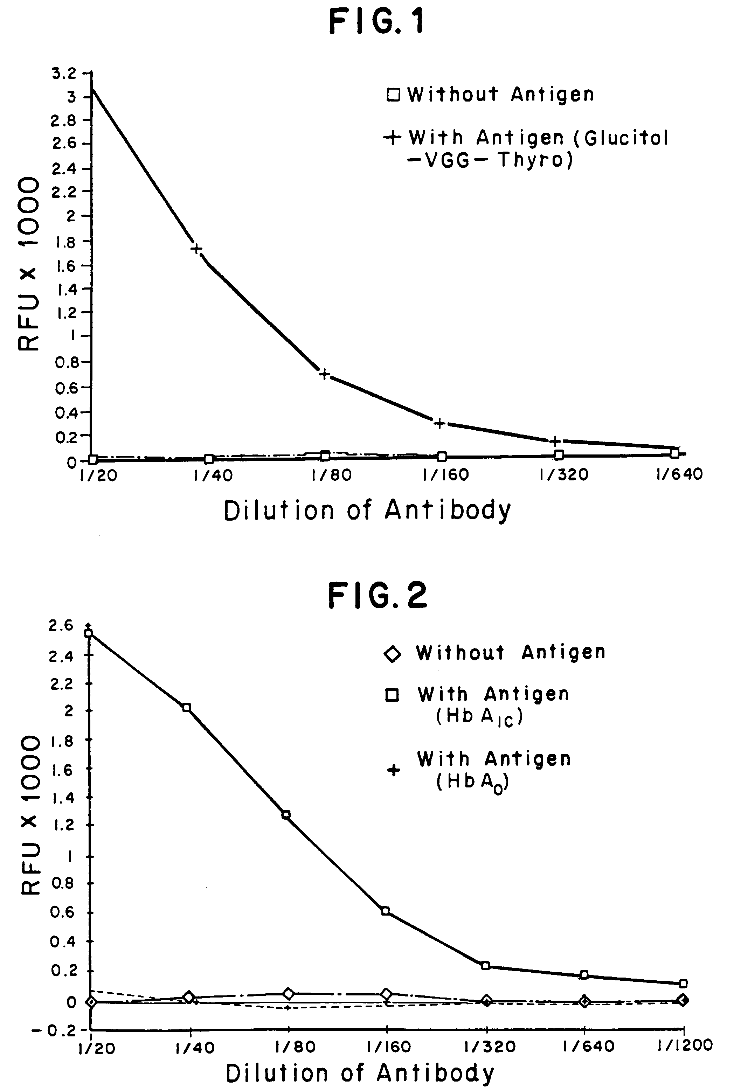

Representative direct binding ELFA results are shown in Figures 1-3 and 5-7. The results may be summarized as follows.

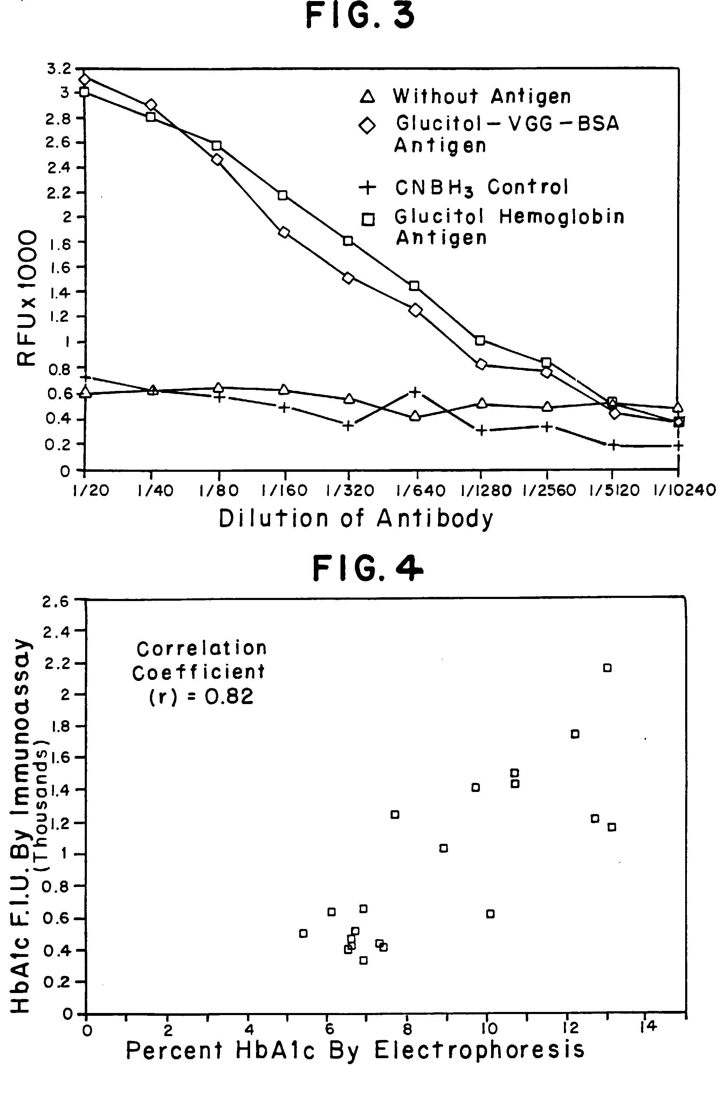

Antibodies prepared against glucitol-VGG-BSA and affinity purified using glucitol-VGG Sepharose recognized glucitol-VGG-BSA and glucitol-VGG-Thyro (Figures 1 and 3). These antibodies also reacted with reduced glycosylated native hemoglobin (Figure 3) and reduced purified HbA_{1c} (Figure 2), but did not react with reduced native hemoglobin (HbAₒ in Figure 2 and CNBH₃ control (hemoglobin reductively glycated in the absence of glucose in Figure 3). The HbAₒ used was prepared by ion exchange chromatography and would be expected to contain molecules glycosylated on available epsilon amino groups which are reduced by the NaBH₄ treatment.

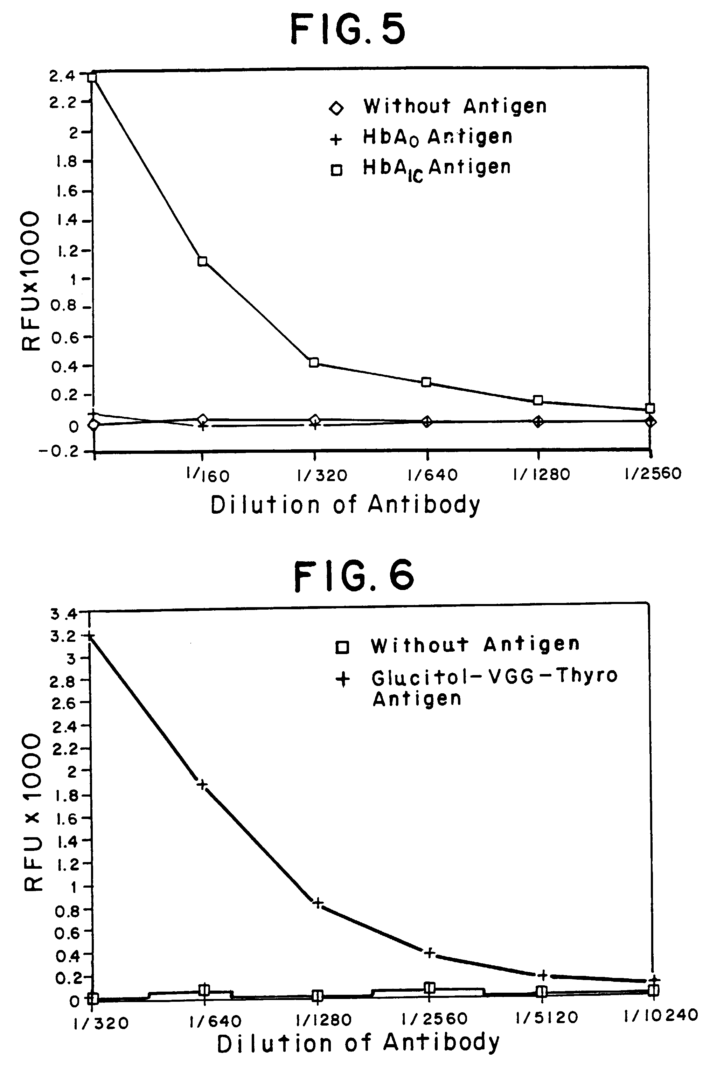

Antibodies prepared using glucitol-VGG-BSA and affinity purified with columns of glucitol-valine-Sepharose showed the same specificities as antibodies purified on glucitol-VGG Sepharose (compare Figure 2 with Figure 5). Similarly, antibodies prepared against glucitol-VGG-BSA and affinity adsorbed using cross-linked human hemoglobin (which could be expected to contain unreduced glycosylated valine and lysine residues) showed the same specificities as antibodies that had been affinity purified with either glucitol-VGG Sepharose or glucitol-valine Sepharose. See Figures 1-3 and 5-7.

Finally, direct binding ELFA results using RBC lysates from diabetic patients correlated well with the measurement of HbA_{1c} obtained independently using a standard electrophoresis assay. See Figure 4 which is a typical regression analysis curve.

The electrophoresis assay was performed using a Ciba-Corning electrophoresis kit for the measurement of glycosylated hemoglobin (Ciba-Corning, Palo Alto, Ca., catalog no. 470055). The electrophoresis assay was performed according to the manufacturer's instructions.

### EXAMPLE 11: Inhibition Enzyme Linked Fluorescence Assay ("Inhibition ELFA")

Inhibition ELFA was performed in the same manner as direct binding ELFA of Example 10 using solid-phase adsorbed reduced Hb_{1c} antigen with two alterations. First, a constant concentration was employed of anti-glucitol-VGG-BSA antibody affinity purified on glucitol-VGG Sepharose. The concentration used was the concentration of antibody required to obtain 50% or less of maximal binding in direct binding ELFA with solid phase adsorbed, NaBH₄-reduced HbA_{1c} antigen.

Second, antibody of fixed concentration was mixed with varying concentrations of inhibitor consisting of Hb_{1c} antigen or lysates of RBC's from diabetic patients, both of which had previously been reduced as described in Example 9 and then serially diluted in PSBA prior to the addition of antibody. After a 2 hour incubation at 37°C, 200 ul of the antibody-inhibitor mixture were added to wells that had been coated with HbA_{1c}, reduced with NaBH₄, washed with PBSA and blocked with ovalbumin as described in Examples 9 and 10. The addition of the antibody-inhibitor mixture in inhibition binding ELFA corresponds to the addition of anti-glucitol-VGG-BSA antibody preparation in direct binding ELFA.

Controls for inhibition ELFA were without antigen controls, without primary antibody controls, substrate blank and controls for the effect of NaBH₄ reduction consisting of anti and diluted reducing mixture which had been treated identically to liquid-phase reduced HbA_{1c} inhibitor, but which lacked inhibitor.

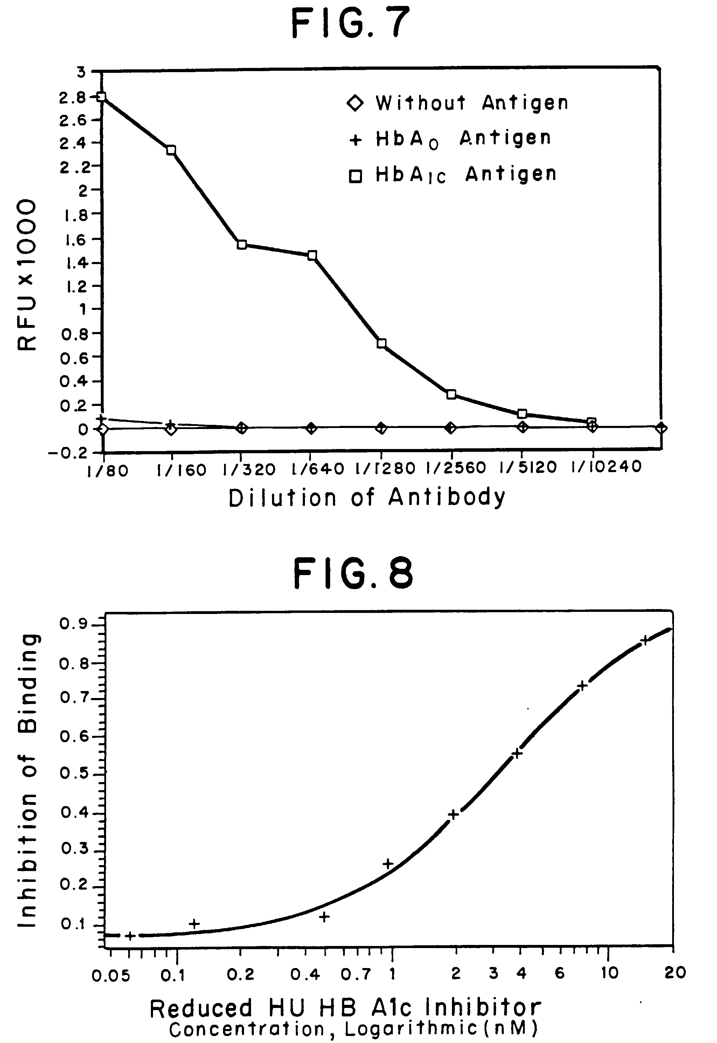

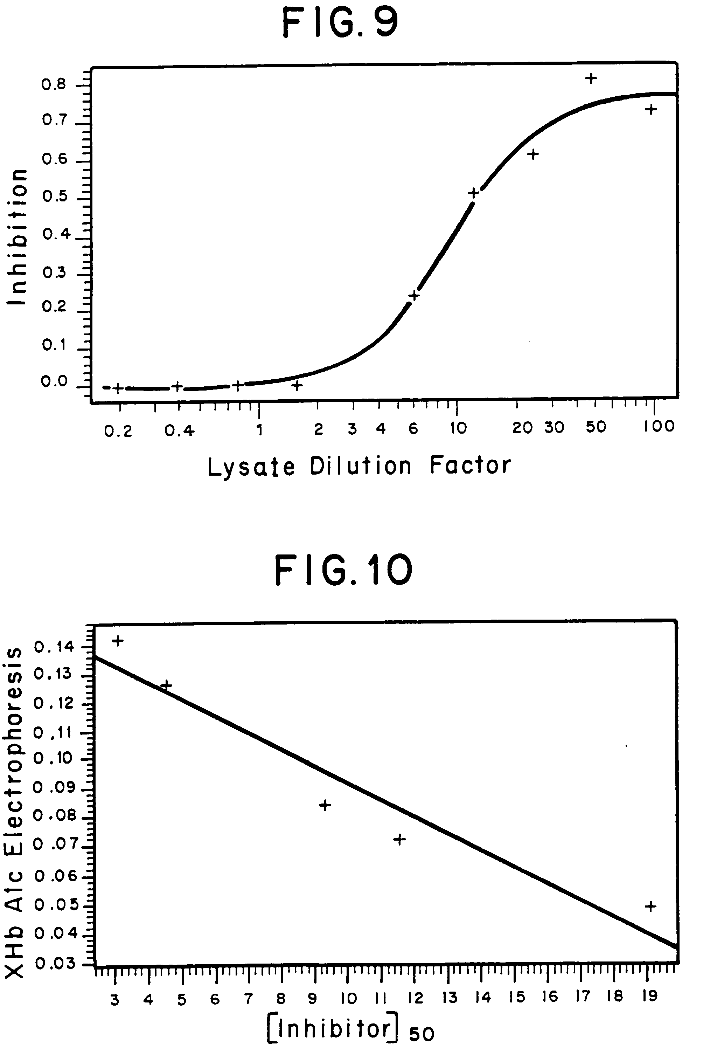

Representative inhibition of antibody binding is shown in Figure 8 for reduced liquid phase HbA_{1c} and in Figure 9 for a reduced RBC lysate sample from a diabetic patient. The RBC lysate had previously been shown by electrophoresis to contain 8.4% HbA_{1c}. Regression analysis demonstrates a linear correlation between the amounts of RBC lysate required to obtain 50% inhibition in inhibition ELFA and the amount of HbA_{1c} determined by electrophoresis to be present in that sample for the five samples studied (see Figure 10).

### EXAMPLE 12: Inhibition Enzyme Linked Fluorescence Assay ("Inhibition ELFA")

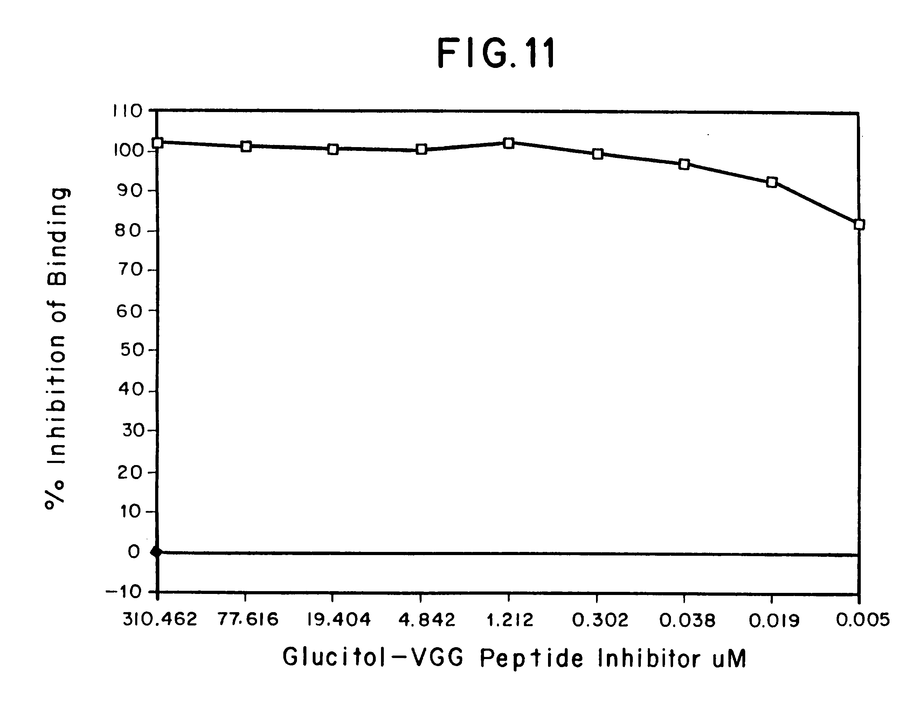

Example 11 was repeated, except that anti-glucitol-VGG-BSA antibody absorbed with cross-linked human hemoglobin was used as the antibody and glucitol-VGG was used as the inhibitor. The results are shown in Figure 11. As can be seen there, glucitol-VGG inhibited 100% of the binding of the antibody to reduced HbA_{1c} at the higher concentrations of glucitol-VGG tested.

The data from Examples 10-12 taken together demonstrate that anti-glucitol-VGG-BSA antibody which has been affinity purified with glucitol-VGG Sepharose or glucitol-valine Sepharose or affinity absorbed with cross-linked human hemoglobin specifically detects glucitol-valine. The affinity purified or affinity absorbed antibody still has a high titer. Thus, the method of the invention provides an easy way to obtain highly specific, high-titered antisera and antibody preparations useful in immunoassays to detect proteins glycosylated on their N-terminal amino acid without the preparation of monoclonal antibodies. Of course, monoclonal antibodies may be used, if desired.

### EXAMPLE 13: Direct Binding Enzyme Linked immunosorbent Assay (ELISA) Using Whole Blood Lysates.

This example describes a direct binding assay which is an adaptation of the method described in Example 10. Whole blood is utilized as a source of HbA_{1c}, and a readily visualized colorimetric endpoint is employed. The complete assay can be performed in a total of about 3-4 hours.

One drop of blood previously collected in EDTA coated vacutainer tubes (Becton Dickinson, Rutherford, NJ 07070) was added to 1 ml of distilled water in a 12 x 75 mm polystyrene tube (Scientific Products McGaw Park, IL, USA) with a 5.75 inch Pasteur-type Dispo Pipet (Scientific Products, McGaw Park, IL, USA). After mixing, the tube was coated with the resulting whole blood lysate by incubating for 15 min. at room temperature. The coating solution was removed by aspiration, and one ml of 50 NaBH₄ in PBS was added, and reduction was allowed to proceed for 10 min. at room temperature.

The tube was then washed three times with PBS. Washing consisted of filling the tube with PBS and then aspirating the wash buffer.

Anti-glucitol-valine reactive antibody (prepared as described in Example 7, except that it was not affinity purified or affinity absorbed, i.e., a DE-52 purified IgG fraction), diluted 1:100 in PBSA was added (1 ml per tube) and allowed to incubate for 45 min. at room temperature.

Following three washes with PBS, one ml of biotin-labeled goat anti-rabbit IgG (Vector Laboratories, Burlingame, CA, USA) diluted 1:2000 in PBSA was added and allowed to incubate for 0.5 hour at room temperature. After three PBS washes, one ml of streptavidin-beta-galactosidase (Bethesda Research Laboratories, Gaithersburg, MD, USA) diluted 1:2000 in PBSA was added and allowed to incubate for 15 min. at room temperature. Following four PBS washes, 1 ml of a 4 mg/ml solution of o-nitrophenyl-beta-D-galactopyranoside (ONPG) substrate (Sigma Chemical Co., St. Louis, MO, USA) in 0.1 M sodium phosphate buffer, pH 7.3, containing 0.1 M 2-mercaptoethanol and 5 mM MgCl₂ was added and allowed to incubate 40 minutes at room temperture. Using standards containing known amounts of HbA_{1c} (Bio-Rad Hemoglobin A_{1c} Mini and/or Micro Column Test Calibrators, Bio-Rad Clinical

Division, Hercules, CA, USA), the relative amounts of HbA_{1c} in whole blood lysates could be determined by a comparison between standards and whole blood lysates of yellow nitro-phenol product obtained.

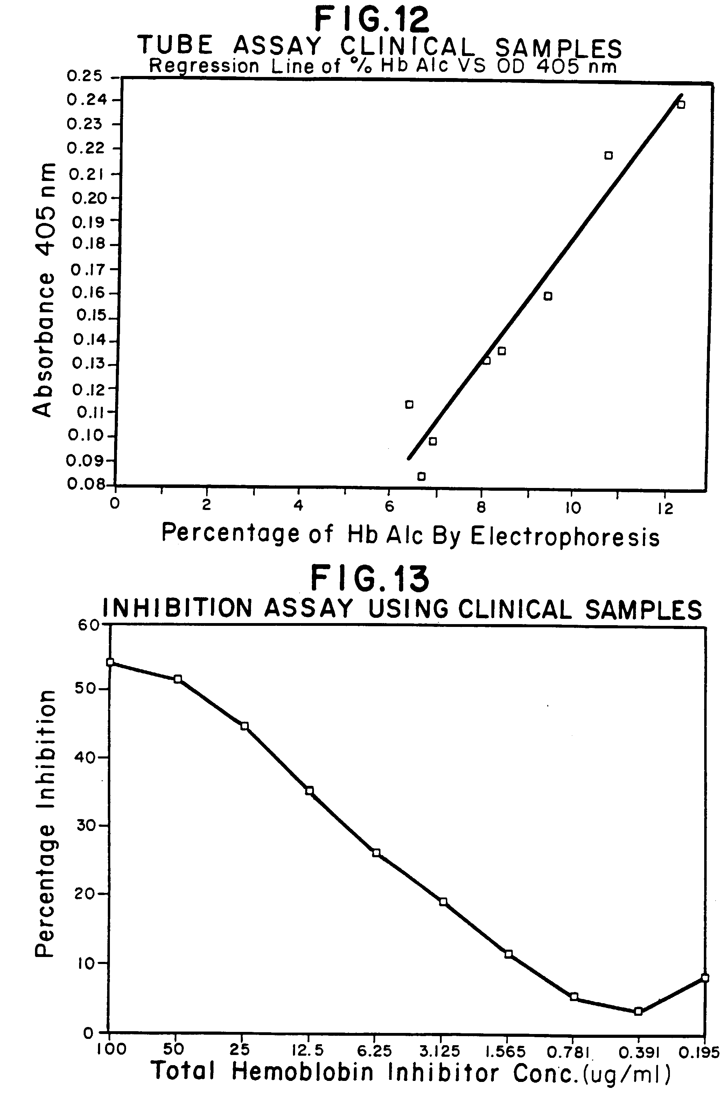

Results obtained using this method to measure the amount of HbA_{1c} in whole blood lysates from diabetic patients correlated well with the percentage of HbA_{1c} obtained by electrophoresis of RBC lysates from the same patients as shown in Figure 12 (correlation coefficient = 0.9724). The electrophoresis assay was performed as described in Example 10.

### EXAMPLE 14: Inhibition Enzyme Linked Fluorescence Assay Using Whole Blood Lysates as Inhibitors.

This example describes an adaptation of the inhibition assay format described in Example 11. Whole blood lysates were used as the source of HbA_{1c}.

Two hundred microliters of antigen (Bio-Rad Hemoglobin A_{1c} Micro Column Test Standard assayed to contain 11.4% HbA_{1c}) was added to Microfluor "B" microtiter wells at a concentration of 10µg/ml of total hemoglobin in 0.1 M NaHCO₃, pH 9.8, and allowed to coat the wells for 2 hours at 37°C. Unbound antigen was removed by decanting or aspirating, and adsorbed antigen was reduced by filling the wells with 50 mM NaBH₄ in PBS and incubating for 1 hour at 37°C. Wells were subsequently washed 5 times with PBS containing 0.1% Tween 20 (Sigma Chemical Co., St. Louis, MO, USA) and filled with PBS containing 1% ovalbumin (Sigma Chemical Co., St. Louis, MO, USA) and 0.02% sodium azide, pH 7.3, and allowed to incubate for 1 hour at 37°C to block any remaining polystyrene protein binding sites.

During the coating, reducing and backcoating steps, anti-glucitol-valine reactive antibody, prepared as described in Example 13, was incubated with NaBH₄-reduced inhibitors (either whole blood lysates or standards consisting of Bio-Rad Hemoglobin A_{1c} calibrators, Bio-Rad Clinical Division, Hercules, CA, USA). Reduction of these inhibitors was performed as described in Example 9 for RBC lysates, except that the concentration of NaBH₄ in the reducing solution was 500 mM and the reduction period was 1 hour at 37°C.

Subsequent steps of the inhibition assay were identical to those described in Example 11, with the following exceptions:
(1) Washes were done with PBS containing 0.1% Tween 20; and
(2) The incubation period with biotin-labeled goat anti-rabbit IgG was overnight at 4°C.

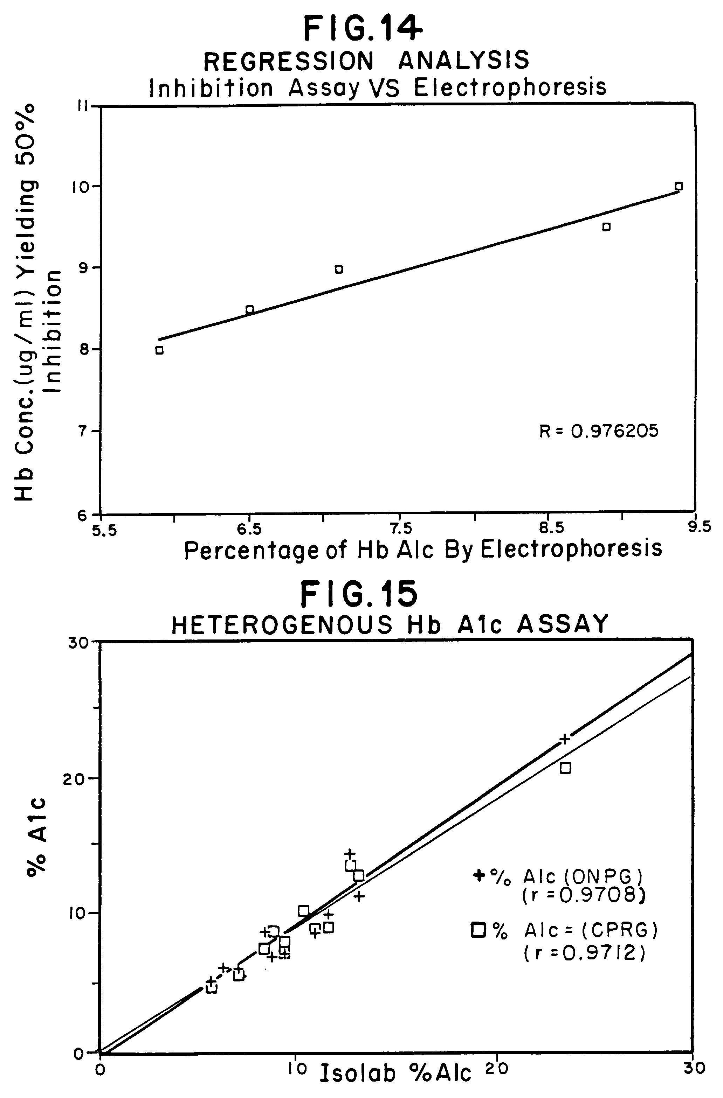

A representative dose response curve showing inhibition of antibody binding using a diabetic whole blood lysate as inhibitor is shown in Figure 13. Regression analysis was performed comparing the results of measuring the amount of HbA_{1c} in whole blood lysates from diabetics by the described inhibition method with the results obtained by electrophoresis of RBC lysates from the same patients (electrophoresis was done as described in Example 10). A graph of the regression analysis results comparing the two techniques for 5 of the 8 diabetic samples tested are shown in Figure 14. The correlation coefficient for these five samples was 0.9762. Regression analysis of 6 of the 8, 7 of the 8, and 8 of the 8 samples gave, respectively, correlation coefficients of 0.7098, 0.4899 and 0.0774. It is not yet known why these three samples gave such divergent results.

In Figures 13 and 14, the hemoglobin concentration of the whole blood lysates was determined by comparison of the absorbance of the lysates at 414 nm with the absorbance of a serially diluted hemoglobin standard (Sigma Chemical Co., St. Louis, MO; USA) dissolved and diluted in distilled water.

### EXAMPLE 15: Direct Binding Enzyme Linked Immunosorbent Assay (ELISA) Using Whole Blood Lysates.

Direct binding ELISA using whole blood lysates as a source of HbA_{1c} was performed using microtiter plates. The complete assay can be performed in a total of about 3-4 hours.

Blood samples were lysed by dilution to 1:200 in distilled water, and the hemoglobin concentration was subsequently adjusted to 10 µg/ml in 0.1M NaHCO₃, pH 9.6 ("coating buffer"). Hemoglobin concentrations were determined by comparison of the absorbance of the lysates at 414 nm with the absorbance of a serially diluted hemoglobin standard (Sigma Chemical Co., St. Louis, MO; USA) dissolved and diluted in distilled water. Standards containing known amounts of hemoglobin A_{1c} (Bio-Rad Hemoglobin A_{1c} Mini and/or Micro Column Test Calibrators, Bio-Rad Clinical Division, Hercules, CA, USA) were diluted to 10 µg/ml in coating buffer.

Two hundred microliters of antigen (i.e. diluted diabetic whole blood lysates or diluted standards containing known amounts of hemoglobin A_{1c}) were added to wells of Immulon 2 plates (Dynatech Laboratories, Inc., Chantilly, VA, USA). The plates were incubated for 1 hour at 37°C to allow the antigen to adsorb to the solid surfaces of the wells. The antigens were added at a coating concentration of 10 µg/ml in coating buffer.

Subsequent steps were identical to those used in the direct binding ELFA described in Example 10, with the following exceptions:
1. Reduction with 50 mM NaBH₄ in PBS, pH 7.3, was for 0.5 hours at room temperature.
2. Incubation with anti-glucitol-valine antibody (prepared as described in Example 13) was for 45 minutes at 37°C using a 1/20 dilution of antibody;
3. Incubation with biotin-labeled goat anti-rabbit IgG was for 45 minutes at 37°C using a 1/2000 dilution of antibody;
4. Incubation with streptavidin-β-galactosidase was for 20 minutes at room temperature;
5. All washes between addition of reagents were performed in triplicate using PBS containing 0.1% Tween 20, pH 7.3;
6. Substrate was either: a) o-nitrophenyl-β-D-galactopyranoside ("ONPG") (Sigma Chemical Co., St. Louis, MO, USA) at a concentration of 1 mg/ml in 0.1 M sodium phosphate buffer, pH 7.3, containing 0.1 M 2-mercaptoethanol and 5 mM MgCl₂ ("colorimetric substrate buffer"); or 2) chlorophenol red-β-D-galactopyranoside ("CPRG") (Boehringer Mannheim Biochemicals, Indianapolis, IN, USA) at a concentration of 0.6 mg/ml in colorimetric substrate buffer; and
7. After 40 minutes reaction with ONPG substrate, absorbance at 405 nm was measured. For CPRG, the reaction time was 9 minutes, and absorbance at 570 nm was measured.

Figure 15 shows a comparison of the results obtained by measuring HbA_{1c} in whole blood lysates from diabetics using the direct binding ELISA with those obtained using an aminophenyl boronate system (Glyc-Aff-Ghb, Isolab Inc., Akron, OH, USA), performed according to the manufacturer's instructions, to measure HbA_{1c} in RBC lysates from the same patients. The percentages of HbA_{1c} obtained using the direct binding ELISA and those obtained using the Isolab aminophenyl boronate column system were similar for both ONPG (correlation coefficient = 0.9708) and CPRG (correlation coefficient = 0.9712) substrates. See Figure 15.

## Claims (Claims for the following Contracting State(s): AT, BE, CH, LI, DE, DK, FR, GB, IT, LU, NL, SE)

1. An immunoassay for a protein that is non-enzymatically glycosylated on the alpha amino group of its N-terminal amino acid, the immunoassay comprising:
- providing a sample containing the glycosylated protein;
- reacting the glycosylated protein with a reducing agent so that the sugar residue on the N-terminal amino acid is reduced;
- contacting the reduced glycosylated protein with an antibody directed to Glc-ol-X wherein X is the N-terminal amino acid of the glycosylated protein, except that X cannot be lysine, and Glc-ol is the reduced form of the sugar residue attached to the alpha amino group of X on the glycosylated protein; and
- detecting or quantitating the reduced glycosylated protein bound to the antibody.

2. The immunoassay of claim 1 wherein X is valine.

3. The immunoassay of claim 1 wherein the protein is glycosylated with glucose or a derivative.

4. The immunoassay of claim 3 wherein the glycosylated protein is hemoglobin.

5. The immunoassay of claim 4 wherein the glycosylated protein is HbA_{1c}, Glc-ol is glucitol and X is valine.

6. The immunoassay of claim 5 wherein the sample is whole blood which has been lysed to release HbA_{1c}.

7. The immunoassay of claim 6 wherein the HbA_{1c} is detected or quantitated colorimetrically.

8. The immunoassay of claim 6 further comprising the step of coating a solid surface with the released HbA_{1c}.

9. The immunoassay of claim 8 wherein the reduced HbA_{1c} is detected or quantitated colorimetrically.

10. Antibody directed to Glc-ol-X specifically adapted for use in the immunoassay according to claims 1 to 9, wherein X is valine and Glc-ol is the reduced form of the sugar residue attached to the alpha amino group of valine on the glycolysated protein.

11. The antibody of claim 10, which is directed to glucitol-valine.

12. Immunogen specifically designated for the preparation of the antibody directed to Glc-ol-X according to claim 10, having the formula
(Glc-ol-X-L)ₙ-carrier
wherein
X is valine;
L is a bond or a linker group;
Glc-ol is the reduced form of the sugar attached to the alpha amino group of valine on the glycolysated protein;
the carrier is an immunogenic compound other than the glycosylated protein; and
n is from 1 to the number of available coupling sites on the carrier.

13. The immunogen of claim 12 wherein Glc-ol is glucitol.

14. The immunogen of claim 12 wherein L is glycine-glycine.

15. The immunogen of claim 12 wherein the carrier is bovine serum albumin or bovine thyroglobin.

16. The immunogen of claim 13 wherein L is glycine-glycine.

17. The immunogen of claim 16 wherein the carrier is bovine serum albumin or bovine thyroglobin.

18. A method of preparing the antibody directed to Glc-ol-X according to claim 10, the method comprising immunizing an animal with the immunogen according to claim 12.

19. The method of claim 18 wherein the protein is glycosylated with glucose or a derivative.

20. The method of claim 19 wherein the glycosylated protein is hemoglobin.

21. The method of claim 20 wherein the glycosylated protein is HbA_{1c} and Glc-ol is glucitol.

22. The method of claim 21 wherein L is glycine-glycine.

23. The method of claim 22 wherein the carrier is bovine serum albumin or bovine thyroglobin.

24. The method of claim 18 wherein L is glycine-glycine.

25. A method of preparing the immunogen of claim 12, comprising:
- reductively glycosylating X to produce Glc-ol-X;
- coupling the Glc-ol-X to L if a linker group is to be used; and
- coupling Glc-ol-X or Glc-ol-X-L to an immunogen carrier.

26. The method of claim 25 wherein Glc-ol is glucitol and L is glycine-glycine.

27. A method of preparing the immunogen of claim 12, comprising:
- coupling X to L to produce X-L;
- reductively glycosylating X-L to produce Glc-ol-X-L;
- coupling the Glc-ol-X-L to an immunogenic carrier.

28. The method of claim 27 wherein Glc-ol is glucitol and L is glycine-glycine.

29. A kit for the immunoassay according to claim 1, comprising:
a container of a first antibody directed to Glc-ol-X, wherein X is valine and Glc-ol is the reduced form of the sugar attached to valine on the glycosylated protein.

30. The kit of claim 29 further comprising a container of reducing agent for reducing the sugar residue on the alpha amino group of X.

31. The kit of claim 30 further comprising a container of a labeled component useful for detecting or quantitating reduced glycosylated protein bound to the antibody.

32. The kit of claim 31, wherein the labeled component is a second antibody reactive with the first antibody.

33. The kit of claim 29 further comprising a container of a labeled component useful for detecting or quantitating reduced glycosalated protein bound to the antibody.

34. The kit of claim 33 wherein the labeled component is a second antibody reactive with the first antibody.

## Claims (Claims for the following Contracting State(s): ES)

1. An immunoassay for a protein that is non-enzymatically glycosylated on the alpha amino group of its N-terminal amino acid, the immunoassay comprising:
- providing a sample containing the glycosylated protein;
- reacting the glycosylated protein with a reducing agent so that the sugar residue on the N-terminal amino acid is reduced;
- contacting the reduced glycosylated protein with an antibody directed to Glc-ol-X wherein X is the N-terminal amino acid of the glycosylated protein, except that X cannot be lysine, and Glc-ol is the reduced form of the sugar residue attached to the alpha amino group of X on the glycosylated protein; and
- detecting or quantitating the reduced glycosylated protein bound to the antibody.

2. The immunoassay of claim 1 wherein X is valine.

3. The immunoassay of claim 1 wherein the protein is glycosylated with glucose or a derivative.

4. The immunoassay of claim 3 wherein the glycosylated protein is hemoglobin.

5. The immunoassay of claim 4 wherein the glycosylated protein is HbA_{1c}, Glc-ol is glucitol and X is valine.

6. The immunoassay of claim 5 wherein the sample is whole blood which has been lysed to release HbA_{1c}.

7. The immunoassay of claim 6 wherein the HbA_{1c} is detected or quantitated colorimetrically.

8. The immunoassay of claim 6 further comprising the step of coating a solid surface with the released HbA_{1c}.

9. The immunoassay of claim 8 wherein the reduced HbA_{1c} is detected or quantitated colorimetrically.

10. A method of preparing an immunogen specifically designated for the preparation of an antibody directed to Glc-ol-X specifically adapted for use in the immunoassay according to claims 1 to 9 wherein X is valine, the immunogen having the formula
(Glc-ol-X-L)ₙ-carrier
wherein
X is valine;
L is a bond or a linker group;
Glc-ol is the reduced form of the sugar attached to the alpha amino group of valine on the glycolysated protein;
the carrier is an immunogenic compound other than the glycosylated protein; and
n is from 1 to the number of available coupling sites on the carrier,
the method comprising:
- reductively glycosylating X to produce Glc-ol-X;
- coupling the Glc-ol-X to L if a linker group is to be used; and
- coupling Glc-ol-X or Glc-ol-X-L to an immunogen carrier.

11. The method of claim 10 wherein Glc-ol is glucitol.

12. The method of claim 10 wherein L is glycine-glycine.

13. The method of claim 10 wherein the carrier is bovine serum albumin or bovine thyroglobin.

14. The method of claim 11 wherein L is glycine-glycine.

15. The method of claim 14 wherein the carrier is bovine serum albumin or bovine thyroglobin.

16. A method of preparing an immunogen specifically designated for the preparation of an antibody directed to Glc-ol-X specifically adapted for use in the immunoassay according to claims 1 to 9 wherein X is valine, the immunogen having the formula
(Glc-ol-X-L)ₙ-carrier
wherein
X is valine;
L is a bond or a linker group;
Glc-ol is the reduced form of the sugar attached to the alpha amino group of valine on the glycolysated protein;
the carrier is an immunogenic compound other than the glycosylated protein; and
n is from 1 to the number of available coupling sites on the carrier,
the method comprising:
- coupling X to L to produce X-L;
- reductively glycosylating X-L to produce Glc-ol-X-L;
- coupling the Glc-ol-X-L to an immunogenic carrier.

17. The method of claim 16 wherein Glc-ol is glucitol.

18. The method of claim 16 wherein L is glycine-glycine.

19. The method of claim 16 wherein the carrier is bovine serum albumin or bovine thyroglobin.

20. The method of claim 17 wherein L is glycine-glycine.

21. The method of claim 20 wherein the carrier is bovine serum albumin or bovine thyroglobin.

## Patentansprüche (Patentansprüche für folgende(n) Vertragsstaat(en): AT, BE, CH, LI, DE, DK, FR, GB, IT, LU, NL, SE)

1. immuntest für ein Protein, welches an der alpha-Aminogruppe seiner N-Endaminosäure nicht-enzymatisch glykosyliert ist, welcher Immuntest folgendes aufweist:
- Bereitstellen einer das glykosylierte Protein enthaltenden Probe;
- Umsetzen des glykosylierten Proteins mit einem Reduziermittel so, daß der Zuckerrest an der N-Endaminosäure reduziert wird;
- Kontaktieren des reduzierten glykosylierten Proteins mit einem auf Glc-ol-X gerichteten Antikörper, wobei X die N-Endaminosäure des glykosylierten Proteins ist, außer daß X nicht Lysin sein kann, und Glc-ol die reduzierte Form des an der alpha-Aminogruppe von X angehängten Zuckerrestes am glykosylierten Protein ist; und
- Bestimmen oder Quantifizieren des an den Antikörper gebundenen glykosylierten Proteins.

2. Immuntest nach Anspruch 1, bei dem X Valin ist.

3. Immuntest nach Anspruch 1, bei dem das Protein mit Glukose oder einem Derivat glykosyliert ist.

4. Immuntest nach Anspruch 3, bei dem das glykosylierte Protein Hämoglobin ist.

5. Immuntest nach Anspruch 4, bei dem das glykosylierte Protein HbA_{1c}, Glc-ol Glucitol und X Valin ist.

6. Immuntest nach Anspruch 5, bei dem die Probe ganzes Blut ist, das zur Freisetzung von HbA_{1c} lysiert worden ist.

7. Immuntest nach Anspruch 6, bei dem das HbA_{1c} kolorimetrisch bestimmt oder quantifiziert wird.

8. Immuntest nach Anspruch 6, der ferner den Verfahrensschritt der Beschichtung einer festen Fläche mit dem freigesetzten HbA_{1c} umfaßt.

9. Immuntest nach Anspruch 8, bei dem das reduzierte HbA_{1c} kolorimetrisch bestimmt oder quantifiziert wird.

10. Antikörper, welcher auf Glc-ol-X gerichtet und spezifisch für die Verwendung in dem Immuntest nach den Ansprüchen 1 bis 9 angepaßt ist, bei dem X Valin und Glc-ol die reduzierte Form des an der alpha-Aminogruppe von Valin angehängten Zuckerrestes am glykosylierten Protein ist.

11. Antikörper nach Anspruch 10, der auf Glucitol-Valin gerichtet ist.

12. Immunogen, das spezifisch für die Herstellung des auf Glc-ol-X gerichteten Antikörpers nach Anspruch 10 bestimmt ist und die Formel hat:
(Glc-ol-X-L)ₙ-Träger
worin
X Valin ist;
L eine Binde- oder Verbindungsgruppe ist;
Glc-ol die reduzierte Form des an der alpha-Aminogruppe von Valin angehängten Zuckers am glykosylierten Protein ist;
der Träger eine andere immunogene Verbindung als das glykosylierte Protein ist; und
n von 1 bis zur Zahl der verfügbaren Kupplungsstellen am Träger ist.

13. Immunogen nach Anspruch 12, bei dem Glc-ol Glucitol ist.

14. Immunogen nach Anspruch 12, bei dem L Glycin-Glycin ist.

15. Immunogen nach Anspruch 12, bei dem der Träger Rinderserumalbumin oder Rinderthyroglobin ist.

16. Immunogen nach Anspruch 13, bei dem L Glycin-Glycin ist.

17. Immunogen nach Anspruch 16, bei dem der Träger Rinderserumalbumin oder Rinderthyroglobin ist.

18. Verfahren zum Herstellen des auf Glc-ol-X gerichteten Antikörpers nach Anspruch 10, welches Verfahren das Immunisieren eines Tieres mit dem Immunogen nach Anspruch 12 umfaßt.

19. Verfahren nach Anspruch 18, bei dem das Protein mit Glukose oder einem Derivat glykosyliert ist.

20. Verfahren nach Anspruch 19, bei dem das glykosylierte Protein Hämoglobin ist.

21. Verfahren nach Anspruch 20, bei dem das glykosylierte Protein HbA_{1c} und Glc-ol Glucitol ist.

22. Verfahren nach Anspruch 21, bei dem L Glycin-Glycin ist.

23. Verfahren nach Anspruch 22, bei dem der Träger Rinderserumalbumin oder Rinderthyroglobin ist.

24. Verfahren nach Anspruch 18, bei dem L Glycin-Glycin ist.

25. Verfahren zum Herstellen des Immunogens nach Anspruch 12, welches folgendes umfaßt:
- reduzierendes Glykosylieren von X, um Glc-ol-X herzustellen;
- Kuppeln von Glc-ol-X an L, falls eine Verbindungsgruppe verwendet werden soll; und
- Kuppeln von Glc-ol-X oder Glc-ol-X-L an einen Immunogenträger.

26. Verfahren nach Anspruch 25, bei dem Glc-ol Glucitol und L Glycin-Glycin ist.

27. Verfahren zum Herstellen des Immunogens nach Anspruch 12, welches folgendes umfaßt:
- Kuppeln von X an L, um X-L herzustellen;
- reduzierendes Glykosylieren von X-L, um Glc-ol-X-L herzustellen;
- Kuppeln von Glc-ol-X-L an einen Immunogenträger.

28. Verfahren nach Anspruch 27, bei dem Glc-ol Glucitol und L Glycin-Glycin ist.

29. Zusammensetzung für einen Immuntest nach Anspruch 1, welche folgendes aufweist:
einen Aufnehmer für einen ersten, auf Glc-ol-X gerichteten Antikörper, bei dem X Valin und Glc-ol die reduzierte Form des an Valin angehängten Zuckers am glykosylierten Protein ist.

30. Zusammensetzung nach Anspruch 29, welche ferner einen Aufnehmer für ein Reduziermittel zum Reduzieren des an der alpha-Aminogruppe von X angehängten Zuckerrestes ist.

31. Zusammensetzung nach Anspruch 30, welche ferner einen Aufnehmer für eine markierte, zum Bestimmen oder Quantifizieren von reduziertem glykosyliertem, an den Antikörper gebundenen Protein zweckmäßige Komponente aufweist.

32. Zusammensetzung nach Anspruch 31, bei dem die markierte Komponente ein zweiter, mit dem ersten Antikörper reagierender Antikörper ist.

33. Zusammensetzung nach Anspruch 29, welche ferner einen Aufnehmer für eine markierte, zum Bestimmen oder Quantifizieren von reduziertem glykosyliertem, an den Antikörper gebundenen Protein zweckmäßige Komponente aufweist.

34. Zusammensetzung nach Anspruch 33, bei dem die markierte Komponente ein zweiter, mit dem ersten Antikörper reagierender Antikörper ist.

## Patentansprüche (Patentansprüche für folgende(n) Vertragsstaat(en): ES)

1. immuntest für ein Protein, welches an der alpha-Aminogruppe seiner N-Endaminosäure nicht-enzymatisch glykosyliert ist, welcher Immuntest folgendes aufweist:
- Bereitstellen einer das glykosylierte Protein enthaltenden Probe;
- Umsetzen des glykosylierten Proteins mit einem Reduziermittel so, daß der Zuckerrest an der N-Endaminosäure reduziert wird;
- Kontaktieren des reduzierten glykosylierten Proteins mit einem auf Glc-ol-X gerichteten Antikörper, wobei X die N-Endaminosäure des glykosylierten Proteins ist, außer daß X nicht Lysin sein kann, und Glc-ol die reduzierte Form des an der alpha-Aminogruppe von X angehängten Zuckerrestes am glykosylierten Protein ist; und
- Bestimmen oder Quantifizieren des an den Antikörper gebundenen glykosylierten Proteins.

2. Immuntest nach Anspruch 1, bei dem X Valin ist.

3. Immuntest nach Anspruch 1, bei dem das Protein mit Glukose oder einem Derivat glykosyliert ist.

4. Immuntest nach Anspruch 3, bei dem das glykosylierte Protein Hämoglobin ist.

5. Immuntest nach Anspruch 4, bei dem das glykosylierte Protein HbA_{1c}, Glc-ol Glucitol und X Valin ist.

6. Immuntest nach Anspruch 5, bei dem die Probe ganzes Blut ist, das zur Freisetzung von HbA_{1c} lysiert worden ist.

7. Immuntest nach Anspruch 6, bei dem das HbA_{1c} kolorimetrisch bestimmt oder quantifiziert wird.

8. Immuntest nach Anspruch 6, der ferner den Verfahrensschritt der Beschichtung einer festen Fläche mit dem freigesetzten HbA_{1c} umfaßt.

9. Immuntest nach Anspruch 8, bei dem das reduzierte HbA_{1c} kolorimetrisch bestimmt oder quantifiziert wird.

10. Verfahren zum Herstellen eines Immunogens, das spezifisch für die Herstellung eines Antikörpers bestimmt ist, welcher auf Glc-ol-X gerichtet und spezifisch für die Verwendung in dem Immuntest nach den Ansprüchen 1 bis 9 angepaßt ist, bei dem X Valin ist, und das Immunogen die Formel hat:
(Glc-ol-X-L)ₙ-Träger
worin
X Valin ist;
L eine Binde- oder Verbindungsgruppe ist;
Glc-ol die reduzierte Form des an der alpha-Aminogruppe von Valin angehängten Zuckers am glykosylierten Protein ist;
der Träger eine andere immunogene Verbindung als das glykosylierte Protein ist; und
n von 1 bis zur Zahl der verfügbaren Kupplungsstellen am Träger ist,
welches Verfahren folgendes umfaßt:
- reduzierendes Glykosylieren von X, um Glc-ol-X herzustellen;
- Kuppeln von Glc-ol-X an L, falls eine Verbindungsgruppe verwendet werden soll; und
- Kuppeln von Glc-ol-X oder Glc-ol-X-L an einen Immunogenträger.

11. Verfahren nach Anspruch 10, bei dem Glc-ol Glucitol ist.

12. Verfahren nach Anspruch 10, bei dem L Glycin-Glycin ist.

13. Verfahren nach Anspruch 10, bei dem der Träger Rinderserumalbumin oder Rinderthyroglobin ist.

14. Verfahren nach Anspruch 11, bei dem L Glycin-Glycin ist.

15. Verfahren nach Anspruch 14, bei dem der Träger Rinderserumalbumin oder Rinderthyroglobin ist.

16. Verfahren zum Herstellen eines Immunogens, das spezifisch für die Herstellung eines Antikörpers bestimmt ist, welcher auf Glc-ol-X gerichtet und spezifisch für die Verwendung in dem Immuntest nach den Ansprüchen 1 bis 9 angepaßt ist, bei dem X Valin ist, und das Immunogen die Formel hat:
(Glc-ol-X-L)ₙ-Träger
worin
X Valin ist;
L eine Binde- oder Verbindungsgruppe ist;
Glc-ol die reduzierte Form des an der alpha-Aminogruppe von Valin angehängten Zuckers am glykosylierten Protein ist;
der Träger eine andere immunogene Verbindung als das glykosylierte Protein ist; und
n von 1 bis zur Zahl der verfügbaren Kupplungsstellen am Träger ist,
welches Verfahren folgendes umfaßt:
- Kuppeln von X an L, um X-L herzustellen;
- reduzierendes Glykosylieren von X-L, um Glc-ol-X-L herzustellen;
- Kuppeln von Glc-ol-X-L an einen Immunogenträger.

17. Verfahren nach Anspruch 16, bei dem Glc-ol Glucitol ist.

18. Verfahren nach Anspruch 16, bei dem L Glycin-Glycin ist.

19. Verfahren nach Anspruch 16, bei dem der Träger Rinderserumalbumin oder Rinderthyroglobin ist.

20. Verfahren nach Anspruch 17, bei dem L Glycin-Glycin ist.

21. Verfahren nach Anspruch 20, bei dem der Träger Rinderserumalbumin oder Rinderthyroglobin ist.

## Revendications (Revendications pour l'(les) Etat(s) contractant(s) suivant(s): AT, BE, CH, LI, DE, DK, FR, GB, IT, LU, NL, SE)

1. Immuno-essai pour une protéine qui est glycosylée de façon non-enzymatique sur le groupe alpha-amino de son acide aminé en position N-terminale, l'immuno-essai comprenant les étapes consistant à:
- fournir un échantillon contenant la protéine glycosylée;
- faire réagir la protéine glycosylée avec un agent réducteur de sorte que le résidu sucre sur l'acide aminé en position N-terminale soit réduit;
- mettre en contact la protéine glycosylée réduite avec un anticorps dirigé contre Glc-ol-X dans lequel X est l'acide aminé en position N-terminale de la protéine glycosylée, sauf que X ne peut pas être la lysine, et Glc-ol est la forme réduite du résidu sucre lié au groupe alpha-amino de X sur la protéine glycosylée; et
- détecter ou quantifier la protéine glycosylée réduite liée à l'anticorps.

2. Immuno-essai selon la revendication 1, où X est la valine.

3. Immuno-essai selon la revendication 1, où la protéine est glycosylée avec du glucose ou un dérivé.

4. Immuno-essai selon la revendication 3, où la protéine glycosylée est l'hémoglobine.

5. Immuno-essai selon la revendication 4, où la protéine glycosylée est la HbA_{1c}, Glc-ol est le glucitol et X est la valine.

6. Immuno-essai selon la revendication 5, où l'échantillon est du sang complet qui a été lysé pour libérer la HbA_{1c}.

7. Immuno-essai selon la revendication 6, où la HbA_{1c} est détectée ou quantifiée par colorimétrie.

8. Immuno-essai selon la revendication 6, comprenant en outre l'étape consistant à recouvrir une surface solide avec la HbA_{1c} libérée.

9. Immuno-essai selon la revendication 8, où la HbA_{1c} réduite est détectée ou quantifiée par colorimétrie.

10. Anticorps dirigé contre Glc-ol-X particulièrement adapté pour une utilisation dans l'immuno-essai selon les revendications 1 à 9, où X est la valine et Glc-ol est la forme réduite du résidu sucre lié au groupe alpha-amino de la valine sur la protéine glycosylée.

11. Anticorps selon la revendication 10, qui est dirigé contre le glucitol-valine.

12. Immunogène particulièrement conçu pour la préparation de l'anticorps dirigé contre Glc-ol-X selon la revendication 10, ayant la formule
(Glc-ol-X-L)ₙ-support
dans laquelle
X est la valine;
L est une liaison ou un groupe de liaison;
Glc-ol est la forme réduite du sucre lié au groupe alpha-amino de la valine sur la protéine glycosylée;
le support est un composé immunogène autre que la protéine glycosylée; et
n est de 1 au nombre de sites de couplage disponibles sur le support.

13. Immunogène selon la revendication 12, où Glc-ol est le glucitol.

14. Immunogène selon la revendication 12, où L est la glycine-glycine.

15. Immunogène selon la revendication 12, où le support est l'albumine sérique bovine ou la thyroglobine bovine.

16. Immunogène selon la revendication 13, où L est la glycine-glycine.

17. Immunogène selon la revendication 16, où le support est l'albumine sérique bovine ou la thyroglobine bovine.

18. Procédé de préparation de l'anticorps dirigé contre Glc-ol-X selon la revendication 10, le procédé comprenant l'étape consistant à immuniser un animal avec l'immunogène selon la revendication 12.

19. Procédé selon la revendication 18, dans lequel la protéine est glycosylée avec du glucose ou un dérivé.

20. Procédé selon la revendication 19, dans lequel la protéine glycosylée est l'hémoglobine.

21. Procédé selon la revendication 20, dans lequel la protéine glycosylée est la HbA_{1c} et Glc-ol est le glucitol.

22. Procédé selon la revendication 21, dans lequel L est la glycine-glycine.

23. Procédé selon la revendication 22, dans lequel le support est l'albumine sérique bovine ou la thyroglobine bovine.

24. Procédé selon la revendication 18, dans lequel L est la glycine-glycine.

25. Procédé de préparation de l'immunogène de la revendication 12, comprenant les étapes consistant à:
- glycosyler de manière réductive X de manière à produire Glc-ol-X;
- coupler le Glc-ol-X à L si un groupe de liaison doit être utilisé; et
- coupler Glc-ol-X ou Glc-ol-X-L à un support immunogène.

26. Procédé selon la revendication 25, dans lequel Glc-ol est le glucitol et L est la glycine-glycine.

27. Procédé de préparation de l'immunogène de la revendication 12, comprenant les étapes consistant à:
- coupler X à L pour produire X-L;
- glycosyler de manière réductive X-L pour produire Glc-ol-X-L;
- coupler Glc-ol-X-L à un support immunogène.

28. Procédé selon la revendication 27, dans lequel Glc-ol est le glucitol et L est la glycine-glycine.

29. Trousse pour l'immuno-essai selon la revendication 1, comprenant:
un récipient contenant un premier anticorps dirigé contre Glc-ol-X, dans lequel X est la valine et Glc-ol est la forme réduite du sucre lié à la valine sur la protéine glycosylée.

30. Trousse selon la revendication 29, comprenant en outre un récipient contenant un agent réducteur pour réduire le résidu sucre sur le groupe alpha-amino de X.

31. Trousse selon la revendication 30, comprenant en outre un récipient contenant un constituant marqué utile pour détecter ou quantifier la protéine glycosylée réduite liée à l'anticorps.

32. Trousse selon la revendication 31, dans laquelle le constituant marqué est un second anticorps réactif avec le premier anticorps.

33. Trousse selon la revendication 29, comprenant en outre un récipient contenant un constituant marqué utile pour détecter ou quantifier la protéine glycosylée réduite liée à l'anticorps.

34. Trousse selon la revendication 33, dans laquelle le constituant marqué est un second anticorps réactif avec le premier anticorps.

## Revendications (Revendications pour l'(les) Etat(s) contractant(s) suivant(s): ES)

1. Immuno-essai pour une protéine qui est glycosylée de façon non-enzymatique sur le groupe alpha-amino de son acide aminé en position N-terminale, l'immuno-essai comprenant les étapes consistant à:
- fournir un échantillon contenant la protéine glycosylée;
- faire réagir la protéine glycosylée avec un agent réducteur de sorte que le résidu sucre sur l'acide aminé en position N-terminale soit réduit;
- mettre en contact la protéine glycosylée réduite avec un anticorps dirigé contre Glc-ol-X dans lequel X est l'acide aminé en position N-terminale de la protéine glycosylée, sauf que X ne peut pas être la lysine, et Glc-ol est la forme réduite du résidu sucre lié au groupe alpha-amino de X sur la protéine glycosylée; et
- détecter ou quantifier la protéine glycosylée réduite liée à l'anticorps.

2. Immuno-essai selon la revendication 1, où X est la valine.

3. Immuno-essai selon la revendication 1, où la protéine est glycosylée avec du glucose ou un dérivé.

4. Immuno-essai selon la revendication 3, où la protéine glycosylée est l'hémoglobine.

5. Immuno-essai selon la revendication 4, où la protéine glycosylée est la HbA_{1c}, Glc-ol est le glucitol et X est la valine.

6. Immuno-essai selon la revendication 5, où l'échantillon est du sang complet qui a été lysé pour libérer la HbA_{1c}.

7. Immuno-essai selon la revendication 6, où la HbA_{1c} est détectée ou quantifiée par colorimétrie.

8. Immuno-essai selon la revendication 6, comprenant en outre l'étape consistant à recouvrir une surface solide avec la HbA_{1c} libérée.

9. Immuno-essai selon la revendication 8, où la HbA_{1c} réduite est détectée ou quantifiée par colorimétrie.

10. Procédé de préparation d'un immunogène particulièrement conçu pour la préparation d'un anticorps dirigé contre Glc-ol-X particulièrement adapté pour une utilisation dans l'immuno-essai selon les revendications 1 à 9 où X est la valine, l'immunogène ayant la formule
(Glc-ol-X-L)ₙ-support
dans laquelle
X est la valine;
L est une liaison ou un groupe de liaison;
Glc-ol est la forme réduite du sucre lié au groupe alpha-amino de la valine sur la protéine glycosylée;
le support est un composé immunogène autre que la protéine glycosylée; et
n est de 1 au nombre de sites de couplage disponibles sur le support,
le procédé comprenant les étapes consistant à:
- glycosyler de manière réductive X de manière à produire Glc-ol-X;
- coupler Glc-ol-X à L si un groupe de liaison doit être utilisé; et
- coupler Glc-ol-X ou Glc-ol-X-L à un support immunogène.

11. Procédé selon la revendication 10, dans lequel Glc-ol est le glucitol.

12. Procédé selon la revendication 10, dans lequel L est la glycine-glycine.

13. Procédé selon la revendication 10, dans lequel le support est l'albumine sérique bovine ou la thyroglobine bovine.

14. Procédé selon la revendication 11, dans lequel L est la glycine-glycine.

15. Procédé selon la revendication 14, dans lequel le support est l'albumine sérique bovine ou la thyroglobine bovine.

16. Procédé de préparation d'un immunogène particulièrement conçu pour la préparation d'un anticorps dirigé contre Glc-ol-X particulièrement adapté pour une utilisation dans l'immuno-essai selon les revendications 1 à 9 où X est la valine, l'immunogène ayant la formule
(Glc-ol-X-L)ₙ-support
dans laquelle
X est la valine;
L est une liaison ou un groupe de liaison;
Glc-ol est la forme réduite du sucre lié au groupe alpha-amino de la valine sur la protéine glycosylée;
le support est un composé immunogène autre que la protéine glycosylée; et
n est de 1 au nombre de sites de couplage disponibles sur le support,
le procédé comprenant les étapes consistant à:
- coupler X à L pour produire X-L;
- glycosyler de manière réductive X-L pour produire Glc-ol-X-L;
- coupler Glc-ol-X-L à un support immunogène.

17. Procédé selon la revendication 16, dans lequel Glc-ol est le glucitol.

18. Procédé selon la revendication 16, dans lequel L est la glycine-glycine.

19. Procédé selon la revendication 16, dans lequel le support est l'albumine sérique bovine ou la thyroglobine bovine.

20. Procédé selon la revendication 17, dans lequel L est la glycine-glycine.

21. Procédé selon la revendication 20, dans lequel le support est l'albumine sérique bovine ou la thyroglobine bovine.
